# EUROPÄISCHE PATENTANMELDUNG

(11) **EP 2 165 697 A1**
(43) Veröffentlichungstag der Anmeldung: **24.03.2010**
(21) Anmeldenummer: 09170977.4
(22) Anmeldetag: 22.09.2009
(51) Int. Cl.: A61K 8/44, A61K 8/46, A61K 8/49, A61K 8/97, A61Q 5/00

(54) **Zusammensetzungen zur Reduktion des Bruches keratinischer Fasern**

(30) Priorität: 23.09.2008 DE 102008048438
(71) Anmelder: Henkel AG & Co. KGaA, 40589 Düsseldorf (DE)
(72) Erfinder: Battermann, Marlene, 21271, Asendorf (DE); Goddinger, Dieter, 25336, Klein Nordende (DE)

(57) **Zusammenfassung**

Die vorliegende Erfindung betrifft kosmetische Zusammensetzungen zur Reduktion des Bruches keratinischer Fasern. Diese Zusammensetzungen enthalten mindestens eine Verbindung der Formel (Bet-I), mindestens Taurin oder ein Taurinderivat, und mindestens einen Wirkstoff erhältlich aus Pflanzen der Gattung Echinacea.

## Beschreibung

Die vorliegende Erfindung betrifft kosmetische Zusammensetzungen zur Reduktion des Bruches keratinischer Fasern. Diese Zusammensetzungen enthalten mindestens eine Verbindung der Formel (Bet-I), mindestens Taurin oder ein Taurinderivat, und mindestens einen Wirkstoff erhältlich aus Pflanzen der Gattung Echinacea.

Nicht zuletzt durch die starke Beanspruchung der Haare, beispielsweise durch das Färben oder Dauerwellen als auch durch die Reinigung der Haare mit Shampoos und durch Umweltbelastungen, nimmt die Bedeutung von Pflegeprodukten mit möglichst lang anhaltender Wirkung zu. Derartige Pflegemittel beeinflussen die natürliche Struktur und die Eigenschaften der Haare. So können anschließend an solche Behandlungen beispielsweise die Naß- und Trockenkämmbarkeit des Haares, der Halt und die Fülle des Haares optimiert sein, sowie die äußere Struktur des Haares geglättet sein, was sich durch einen erhöhten Glanz zeigt, oder die Haare vor erhöhtem Spliß und erhöhtem Haarbruch geschützt sein.

Die bekannten Wirkstoffe können jedoch nicht alle Bedürfnisse in ausreichendem Maße abdecken. Es besteht daher weiterhin ein Bedarf nach Wirkstoffen bzw. Wirkstoffkombinationen für kosmetische Mittel mit guten pflegenden Eigenschaften und guter biologischer Abbaubarkeit. Insbesondere in farbstoff- und/oder elektrolythaltigen Formulierungen besteht Bedarf an zusätzlichen pflegenden Wirkstoffen, die sich problemlos in bekannte Formulierungen einarbeiten lassen. Diese Wirkstoffe sollen in den kosmetischen Zusammensetzungen insbesondere die innere keratinische Struktur stärken und kräftigen und somit dem Bruch der keratinischen Fasern bei üblichen täglichen mechanischen Beanspruchungen, beispielsweise beim Kämmen, Föhnen, Waschen der Fasern, verhindern oder zumindest deutlich spürbar für den Anwender verzögern.

Ein besonderer Einfluss von Zusammensetzungen enthaltend Carnitin und Taurin konnte auf die biologische Haarverdickung festgestellt werden. Das Haar wächst von der Wurzel an gestärkt und mit einem größeren Durchmesser nach, so dass dieser Effekt besonders lang anhaltend ist.

Von Zusammensetzungen, welche Carnitin oder Carnitintartrat enthalten, ist bekannt, dass sie in der Lage sind, die Haarstruktur positiv zu beeinflussen, in dem die speziellen Haarspezifischen Strukturproteine (die Haarkeratine) stimuliert werden.

Weiterhin ist bekannt, dass Zusammensetzungen enthaltend Carnitintartrat und Taurin das Haar in seiner Struktur, seinem Wachstum und seinem Stoffwechsel positiv beeinflusst.

Überraschenderweise wurde nunmehr festgestellt, dass eine kosmetische Zusammensetzung enthaltend
a) mindestens ein Betain der Formel (Bet-I),
b) mindestens Taurin oder ein Taurinderivat und
c) mindestens einen Wirkstoff erhältlich aus Pflanzen der Gattung Echinacea
in einem kosmetischen Träger den Bruch von kosmetischen Fasern deutlich reduziert. Weiterhin wurde überraschenderweise gefunden, dass diese Zusammensetzungen zu einer Restrukturierung der keratinischen Faser führen.

Gegenstand der Erfindung ist daher die Verwendung einer kosmetischen Zusammensetzung zur Reduktion des Bruches keratinischer Fasern, enthaltend in einem kosmetischen Träger
a) mindestens ein Betain der Formel (Bet-I),
b) mindestens Taurin oder ein Taurinderivat und
c) mindestens einen Wirkstoff erhältlich aus Pflanzen der Gattung Echinacea.

Ein weiterer Gegenstand ist die Verwendung einer kosmetischen Zusammensetzung zur Restrukturierung keratinischer Fasern, enthaltend in einem kosmetischen Träger
a) mindestens ein Betain der Formel (Bet-I),
b) mindestens Taurin oder ein Taurinderivat und
c) mindestens einen Wirkstoff erhältlich aus Pflanzen der Gattung Echinacea.

Die Inhaltsstoffe a) bis c) werden nachfolgend detailliert beschrieben. Soweit nachstehend vom Wirkstoffkomplex (A) gesprochen wird, bezieht sich diese Aussage auf die in den zur Reduktion des Bruches keratinischer Fasern erfindungsgemäßen Zusammensetzungen zwingend enthaltenen Inhaltsstoffe a) bis c).

Haarbehandlungsmittel im Sinne der vorliegenden Erfindung sind beispielsweise Haarfärbemittel, Blondiermittel, Haarshampoos, Haarkonditionierer, konditionierende Shampoos, Haarsprays, Haarspülungen, Haarkuren, Haarpackungen, Haar-Tonics, Dauerwell-Fixierlösungen, Haarfärbeshampoos, Haarfärbemittel, Haarfestiger, Haarlegemittel, Haarstyling-Zubereitungen, Fönwell-Lotionen, Schaumfestiger, Haargele, Haarwachse oder deren Kombinationen. Im Hinblick auf die Tatsache, daß der Verbraucher, insbesondere jedoch Männer oft die Anwendung mehrerer unterschiedlicher Mittel und/oder mehrere Anwendungsschritte scheuen, sind erfindungsgemäße Mittel bevorzugt solche Mittel, welche der Verbraucher, insbesondere der Mann ohnehin anwendet. Bevorzugte erfindungsgemäße Mittel sind daher Shampoos, Konditioniermittel oder Haar-Tonics.

Unter dem Bruch keratinischer Fasern versteht sich erfindungsgemäß das Abbrechen makroskopisch sichtbarer Teile von keratinischen Fasern. Dieses Brechen der keratinischen Fasern wird ausgelöst durch wiederholt auftretende mechanische Beanspruchung der Faser. So führt beispielsweise das tägliche, teilweise mehrfache Auskämmen des Haares zum Haarbruch, dem Verlust von ganzen Fasern, welcher insbesondere im unteren, der Haarwurzel entferntesten Teil der Faser, auftritt.

Hiervon zu unterscheiden ist der Spliß, welcher bevorzugt in den Haarspitzen auftritt. Unter Spliß wird das im Spitzenbereich sich aufspaltende Haar verstanden. Gesplißtes Haar ist jedoch kein gebrochenes Haar, wenngleich Spliß, wenn er nicht kosmetisch behandelt wird, ebenfalls zu Haarbruch führen kann.

Unter Kämmbarkeit versteht sich erfindungsgemäß sowohl die Kämmbarkeit der nassen Faser, als auch die Kämmbarkeit der trockenen Faser. Als Maß für die Kämmbarkeit dient die aufgewendete Kämmarbeit oder die aufgewendete Kraft während des Kämmvorganges eines Faserkollektivs. Die Meßparameter können durch den Fachmann sensorisch beurteilt oder durch Messeinrichtungen quantifiziert werden. Als Griff definiert sich die Taktilität eines Faserkollektivs, wobei der Fachmann sensorisch die Parameter Fülle und Geschmeidigkeit des Kollektivs fühlt und bewertet.

Unter Formgebung wird die Fähigkeit verstanden, einem Kollektiv zuvor behandelter keratinhaltiger Fasern, insbesondere menschlicher Haare, eine Formänderung zu verleihen. In der Haarkosmetik wird auch von Frisierbarkeit gesprochen.

Unter einer oxidativen Haarbehandlung wird erfindungsgemäß die Einwirkung eines oxidativen kosmetischen Mittels, enthaltend in einem kosmetischen Träger mindestens ein Oxidationsmittel, auf Haar definiert.

Als kosmetische Träger eignen sich erfindungsgemäß besonders O/W - , W/O - und W/O/W - Emulsionen in Form von Cremes oder Gelen oder auch tensidhaltige schäumende Lösungen, wie beispielsweise Shampoos, Schaumaerosole oder andere Zubereitungen, die insbesondere für die Anwendung auf dem Haar geeignet sind. Es ist aber auch denkbar, die Inhaltsstoffe in eine pulverförmige oder auch tablettenförmige Formulierung zu integrieren, welche vor der Anwendung in Wasser gelöst wird. Die kosmetischen Träger können insbesondere wässrig oder wässrig-alkoholisch sein.

Ein wässriger kosmetischer Träger enthält mindestens 50 Gew.-% Wasser.

Unter wässrig-alkoholischen kosmetischen Trägern sind im Sinne der vorliegenden Erfindung wässrige Lösungen enthaltend 3 bis 70 Gew.-% eines C₁-C₆-Alkohols, insbesondere Methanol, Ethanol bzw. Propanol, Isopropanol, Butanol, Isobutanol, tert.-Butanol, n-Pentanol, iso-Pentanole, n-Hexanol, iso-Hexanole, Glykol, Glycerin, 1,2-Pentandiol, 1,5-Pentandiol, 1,2-Hexandiol oder 1,6-Hexandiol zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Der Inhaltsstoff a) in den Zusammensetzungen zur Reduktion des Bruches von keratinischen Fasern ist ein Betain gemäß der Formel (Bet-I). Unter Betainen sind hier nicht zu verstehen Betaine mit oberflächenaktiven Eigenschaften, wie sie im Kapitel über Tenside oder Emulgatoren beschrieben werden.

Unter einem Betain im Sinne der vorliegenden Erfindung sind Verbindungen zu verstehen, welche gleichzeitig sowohl eine Gruppierung -NR₃⁽⁺⁾ als auch eine Gruppierung -CR₂COO⁽⁻⁾ enthalten sowie Verbindungen, die eine Gruppierung -NR₃⁺ und eine Gruppierung -CH₂OH aufweisen. Insbesondere sind unter den erfindungsgemäßen Betainen solche zu verstehen, welche der Formel (Bet-I) entsprechen.

R¹R²R³N⁺ - (CR⁴R⁵)ₓ - (CR⁶R⁷)_{y} - (CR⁸R⁹)₂ - Y⁻ (Bet-I)

R¹, R², und R³ stehen hier unabhängig voneinander für:
● Wasserstoff,
● einen Methylrest,
● einen C2 - C4 gesättigten oder ungesättigten, verzweigten oder linearen oder cyclischen Kohlenwasserstoffrest

R⁴, R⁵, R⁶, R⁷, R⁸ und R⁹ stehen hier unabhängig voneinander für:
● Wasserstoff,
● -OR¹⁰,
● einen Methylrest, der einen 1H-lmidazolyl-4-Substituenten tragen kann,
wobei R¹⁰ steht für Wasserstoff, -CH₃ oder einen C2 - C4 gesättigten oder ungesättigten, verzweigten oder linearen oder cyclischen Kohlenwasserstoffrest,
x, y und z stehen unabhängig voneinander für eine ganze Zahl von 0 bis 12 mit der Maßgabe, daß mindestens einer der Parameter x, y oder z von 0 verschieden ist und Y steht für COO oder eine Hydroxygruppe in Kombination mit einem physiologisch verträglichen Anion.

Unter Betainen im Sinne der Erfindung sind auch solche Substanzen zu verstehen, bei denen die genannten charakteristischen Gruppen nur bei der gelösten Substanz sowie innerhalb bestimmter pH-Bereiche der Lösung vorliegen.

Erfindungsgemäß können selbstverständlich alle physiologisch verträglichen Salze der erfindungsgemäßen Betaine, insbesondere auch die Mischsalze der Betaine eingesetzt werden. Unter Mischsalzen sind feste Lösungen verschiedener Substanzen zu verstehen. Zur allgemein anerkannten Definition von Mischkristallen als feste Lösungen sei auf die Fachbücher zur anorganischen Chemie oder der Kristallographie verwiesen.

Es kann weiterhin bevorzugt sein, insbesondere organische mehrfunktionelle Carbonsäuren einzusetzen, die neben mindestens einer Carboxygruppe zusätzlich mindestens eine Hydroxygruppe und/oder mindestens eine Aminogruppe aufweisen. Beispiele für diese organischen Carbonsäuren sind Glycolsäure, Milchsäure, Äpfelsäure, Weinsäure oder Citronensäure etc, welche den Genußsäuren zuzurechnen sind. Weiterhin zählen Aminosäuren wie beispielsweise Histidin, Arginin, Lysin, Ornithin, Citrullin etc. zu den mehrfunktionellen organischen Säuren, welche als Mischsalze mit den erfindungsgemäßen Wirkstoffen (Bet-I) eingesetzt werden können. Erfindungsgemäß kann es bevorzugt sein, die Mischsalze in fester Form in die Formulierungen einzuarbeiten. Es ist jedoch selbstverständlich auch möglich, die Mischsalze in Form ihrer einzelnen Komponenten zu verwenden.

Das Mischungsverhältnis der erfindungsgemäßen Mischsalze kann dabei bezogen auf die jeweiligen Molmassen der einzelnen Komponenten (erfindungsgemäßes Betain der Formel (Bet-I) / Mischsalz bildende Substanz) zwischen 1: 50 und 50 : 1, bevorzugt zwischen 10 : 1 und 1 : 10 und ganz besonders bevorzugt zwischen 3 : 1 und 1 : 3 betragen.

Als Beispiele für erfindungsgemäß besonders geeignete Betaine der Formel (Bet-I) sowie deren Mischsalze sind zu nennen: Carnitin, Carnitintartrat, Carnitin Magnesiumcitrat, Acetylcarnitin, 3-O-Lauroyl-L-carnitin-hydrochlorid, 3-O-Octanoyl-L-carnitin-hydrochlorid, 3-O-Palmitoyl-L-carnitin-hydrochlorid, Betalaine, 1,1-Dimethyl-Prolin, Hercynin (Nα,Nα,Nα-Trimethyl-L-histidinium-betain), Ergothionein (Thionein, 2-Mercapto-Nα, Nα, Nα-trimethyl-L-histidinium-betain), Cholin, Cholinchlorid, Cholinbitartrat, Cholindihydrogencitrat und die in der Literatur als Betain bezeichnete Verbindung N,N,N-trimethylglycin. Diese Mischsalze können erfindungsgemäß bevorzugt sein.

Bevorzugt werden Carnitin, Histidin, Cholin, Betain sowie deren Derivate verwendet. Dabei können die erfindungsgemäßen Zusammensetzungen sowohl eine Verbindung gemäß Formel (Bet-I) als auch mehrere, insbesondere zwei, Verbindungen der Formel (Bet-I) enthalten.

Besonders bevorzugte Verbindungen der Formel (Bet-I) als Mischsalze sind die Mischsalze aus: A1) jeweils Carnitin und einer der folgenden Säuren: Fumarsäure, Zitronensäure, Milchsäure, Weinsäure, Histidin, Arginin, Lysin, Ornithin und Citrullin
A2) jeweils Histidin und einer der folgenden Säuren: Fumarsäure, Zitronensäure, Milchsäure, Weinsäure, Histidin, Arginin, Lysin, Ornithin und Citrullin
A3) N,N,N-trimethylglycin (Betain) und einer der folgenden Säuren: Fumarsäure, Zitronensäure, Milchsäure, Weinsäure, Histidin, Arginin, Lysin, Ornithin und Citrullin
A4) Cholin und einer der folgenden Säuren: Fumarsäure, Zitronensäure, Milchsäure, Weinsäure, Histidin, Arginin, Lysin, Ornithin und Citrullin
A5) Dimethylprolin und einer der folgenden Säuren: Fumarsäure, Zitronensäure, Milchsäure, Weinsäure, Histidin, Arginin, Lysin, Ornithin und Citrullin.

Die Betaine der Formel (Bet-I) sind in den erfindungsgemäßen Mitteln bevorzugt in Mengen von 0,001 bis 10 Gew.-%, bezogen auf die gesamte Zubereitung, enthalten. Mengen von 0,01 bis 10 Gew.-% sind besonders bevorzugt, Mengen von 0,01 bis 5 Gew.-% sind in besonderem Maße bevorzugt, und Mengen von 0,1 bis 5 Gew.-% sind ganz besonders bevorzugt.

Der Inhaltsstoff b) der erfindungsgemäßen Zusammensetzung zur Reduktion des Bruches von keratinischen Fasern ist Taurin oder ein Derivat des Taurines. In der vorliegenden Erfindung ist mit Taurin ausschließlich 2-Aminoethansulfonsäure sowie einige weitere explizit genannte Derivate des Taurines zu verstehen. Unter den Derivaten des Taurines werden Taurine verstanden, welche an der Aminogruppe substituiert sein können. Hierbei kann mindestens eines der Wasserstoffatome, zwei oder sogar alle drei der möglichen Wasserstoffatome des inneren Salzes des Taurines unabhängig voneinander durch mindestens eine C1 - C6 Alkylgruppe oder eine C2 - C6 - Hydroxyalkylgruppe substituiert sein. Die C1 bis C4 - Alkylgruppen können dabei gesättigt, ungesättigt, geradkettig oder verzweigt sein. Bevorzugte Alkylsubstituenten sind Methyl, Ethyl, n-Propyl, iso-Propyl, n-Butyl, iso-Butyl, tert-Buty, n-Pentyl, iso-Pentyl, neo-Pentyl oder n-Hexyl.

Bevorzugte Taurinderivate sind N-Monomethyltaurin und N,N-Dimethyltaurin.

Als weitere Taurinderivate werden auch Cysteinsulfinsäure, Taurocholsäure und Hypotaurin genannt.

Taurin und Taurinderivate können selbstverständlich ebenfalls in Form von Mischkristallen vorliegen und verwendet werden, unabhängig davon, ob sie synthetisch hergestellt wurden oder ob sie als Extrakte vorliegen. Besonders bevorzugt werden die Mischsalze im Falle von Taurin und Taurinderivaten ausgewählt aus:
B1) Taurin und einer der folgenden Säuren: Fumarsäure, Zitronensäure, Milchsäure, Weinsäure, Histidin, Arginin, Lysin, Ornithin und Citrullin,
B2) N,N-Dimethyltaurin und einer der folgenden Säuren: Fumarsäure, Zitronensäure, Milchsäure, Weinsäure, Histidin, Arginin, Lysin, Ornithin und Citrullin,
B3) N-Monomethyltaurin und einer der folgenden Säuren: Fumarsäure, Zitronensäure, Milchsäure, Weinsäure, Histidin, Arginin, Lysin, Ornithin und Citrullin.

Taurin oder dessen Derivate sind in den erfindungsgemäßen Mitteln bevorzugt in Mengen von 0,001 bis 10 Gew.-%, bezogen auf die gesamte Zubereitung, enthalten. Mengen von 0,01 bis 10 Gew.-% sind besonders bevorzugt, Mengen von 0,01 bis 5 Gew.-% sind in besonderem Maße bevorzugt, und Mengen von 0,1 bis 5 Gew.-% sind ganz besonders bevorzugt.

Der Inhaltsstoff c) der erfindungsgemäßen Zusammensetzung zur Reduktion des Bruches von keratinischen Fasern ist ein Wirkstoff erhältlich aus Pflanzen der Gattung Echinacea.

Unter einem Wirkstoff, erhältlich aus Pflanzen der Gattung Echinacea, sind erfindungsgemäß die Pflanze selbst, ihre Pflanzenteile, Extrakte und Presssäfte der Sonnenhutgewächse (Echinacea, Synonym: Brauneria NECKER), insbesondere aus Echinacea angustifolia DC, Echinacea paradoxa (NORTON), Echinacea simulata, E. atrorubens, E. tennesiensis, Echinacea strigosa (MCGREGOR), Echinacea laevigata, Echinacea purpurea (L.) Moench und Echinacea pallida (Nutt), sowie aus diesen Extrakten zu gewinnende Aktivsubstanzen zu verstehen. Besonders bevorzugt werden Presssäfte und Extrakte von Sonnenhutgewächsen, insbesondere von Echinacea purpurea (L) MOENCH, eingesetzt. Bevorzugt werden die Presssäfte bzw. Extrakte aus Kraut (den oberirdischen Pflanzenteilen) und/oder Wurzel der Sonnenhutgewächse gewonnen. Bevorzugter Weise werden die Presssäfte mit mechanischer Pressung gewonnen. Insbesondere bevorzugt ist eine Pressung nach dem von der Fa. Flachsmann patentierten Verfahren gemäß EP 0 730 830 B1, auf deren Offenbarung hiermit im vollen Umfang Bezug genommen wird.

Ganz besonders bevorzugt sind Presssäfte aus dem frischen Echinacea purpurea Kraut (Echinacea purpurea Moench herba). Insbesondere werden die Extrakte und/oder Presssäfte in fester Form eingesetzt.

Gemäß einer besonders bevorzugten Ausführungsform ist der Wirkstoff, erhältlich aus Pflanzen der Gattung Echinacea, ausgewählt aus Presssäften und Extrakten, die aus Echinacea purpurea gewonnen werden.

Wirkstoffe, erhältlich aus Pflanzen der Gattung Echinacea, bevorzugt die Presssäfte und/oder Extrakte aus Echinacea sind in den erfindungsgemäßen Mitteln bevorzugt in Mengen von 0,001 bis 10 Gew.-%, bezogen auf die gesamte Zubereitung, enthalten. Mengen von 0,01 bis 10 Gew.-% sind besonders bevorzugt, Mengen von 0,01 bis 5 Gew.-% sind in besonderem Maße bevorzugt, und Mengen von 0,1 bis 5 Gew.-% sind ganz besonders bevorzugt.

Die erfindungsgemäß bevorzugten Zusammensetzungen enthalten gemäß der zuvor beschriebenen Darstellung:
a) mindestens eine Verbindung der Formel (Bet-I),
b) mindestens Taurin oder ein Taurinderivat,
c) und mindestens einen Wirkstoff erhältlich aus Pflanzen der Gattung Echinacea .

Hierbei sind erfindungsgemäß Zusammensetzungen bevorzugt, welche die Verbindung der Gruppe a) in Form eines Mischsalzes enthalten.

Besonders bevorzugte erfindungsgemäße Zusammensetzungen sind beispielsweise die folgenden Zusammensetzungen enthaltend:
Zusammensetzung 1:
   a) Carnitin und Weinsäure,
   b) Taurin und
   c) einen Wirkstoff aus Pflanzen der Gattung Echinacea, insbesondere ausgewählt aus Presssäften und Extrakten, die aus Echinacea purpurea gewonnen werden.
   Zusammensetzung 2:
   a) Carnitin und Arginin,
   b) Taurin und
   c) einen Wirkstoff aus Pflanzen der Gattung Echinacea, insbesondere ausgewählt aus Presssäften und Extrakten, die aus Echinacea purpurea gewonnen werden.
Zusammensetzung 3:
   a) Carnitin und Histidin,
   b) Taurin und Weinsäure und
   c) einen Wirkstoff aus Pflanzen der Gattung Echinacea, insbesondere ausgewählt aus Presssäften und Extrakten, die aus Echinacea purpurea gewonnen werden.
Zusammensetzung 4:
   a) Carnitin und Lysin,
   b) Taurin und Weinsäure und
   c) einen Wirkstoff aus Pflanzen der Gattung Echinacea, insbesondere ausgewählt aus Presssäften und Extrakten, die aus Echinacea purpurea gewonnen werden.
Zusammensetzung 5:
   a) Carnitin und Ornithin,
   b) Taurin und Weinsäure und
   c) einen Wirkstoff aus Pflanzen der Gattung Echinacea, insbesondere ausgewählt aus Presssäften und Extrakten, die aus Echinacea purpurea gewonnen werden.

Obwohl die erfindungsgemäßen Mittel prinzipiell auf dem Haar verbleiben können, werden die Mittel vorzugsweise nach einer Einwirkzeit von 10 Sekunden bis 60 Minuten wieder ausgespült. Dieses Ausspülen kann mit reinem Wasser oder einem marktüblichen Shampoo erfolgen. Einwirkzeiten von 10 Sekunden bis zu 45 Minuten haben sich als ausreichend erwiesen. In vielen Fällen wird nur eine Einwirkzeit von 10 Sekunden bis zu 20 Minuten benötigt. Für eine besonders intensive und lang anhaltende Wirkung kann es jedoch erforderlich sein, dass die Zusammensetzung auf dem Haar verbleibt. In diesem Falle erfolgt das Ausspülen erst nach Stunden oder Tagen mit der nächsten Wäsche der keratinischen Fasern.

Ein weiterer Gegenstand der Erfindung ist daher ein kosmetisches Verfahren zur Reduktion des Bruches keratinischer Fasern, **dadurch gekennzeichnet, dass** eine erfindungsgemäße Zusammensetzung auf die keratinischen Fasern aufgetragen wird und nach einer Einwirkzeit von 10 Sekunden bis zu 60 Minuten wieder ausgespült wird.

Dabei hat es sich als vorteilhaft erwiesen, wenn die erfindungsgemäße Zusammensetzung zur Reduktion des Bruches keratinischer Fasern in einer Menge von mindestens 5 g bis zu 25 g, auf die Fasern aufgetragen wird. Dieses Auftragen erfolgt im allgemeinen bei Raumtemperatur, also etwa 20 bis 25 °C. Aufgetragen wird auf die trockenen oder bereits nassen oder anderweitig vorbehandelten, beispielsweise shampoonierten keratinischen Fasern. Nach dem Auftragen kann wie üblicherweise für Haarbehandlungsmittel weiter verfahren werden. Bevorzugt ist es jedoch, wenn die mit der Zusammensetzung benetzten keratinischen Fasern für einen Zeitraum von 10 Sekunden bis hin zu 60 Minuten, vorzugsweise von 10 Sekunden bis hin zu 45 Minuten, besonders bevorzugt von 10 Sekunden bis hin zu 20 Minuten und höchst bevorzugt von 10 Sekunden bis zu 15 Minuten mit einem Handtuch oder einer Haube, beispielsweise einer PE oder PP - Folie oder einer handelsüblichen Trockenhaube, bedeckt werden. Hierbei entsteht ein Wärme, welche zu einer intensiveren Wirkung der erfindungsgemäßen Zusammensetzung führt. Die Temperatur beträgt etwa 20 bis 50 °C, bevorzugt etwa 25 bis 45 °C, besonders bevorzugt etwa 30 bis 40 °C. Selbstverständlich kann die erhöhte Temperatur nicht nur mit Hilfe einer Kopfbedeckung erzielt werden. Erfindungsgemäß umfasst ist auch die Anwendung eines Föhnes oder einer üblichen Trockenhaube, um die Temperatur zu erhöhen und so eine intensivere Einwirkung der erfindungsgemäßen Zusammensetzung zu erreichen.

Unabhängig von dem genauen Ablauf der Behandlung hat es sich als vorteilhaft erwiesen, die erfindungsgemäßen Mittel bei einer Temperatur von 20 bis 55 °C, insbesondere von 30 bis 40°C, anzuwenden.

Das erfindungsgemäße Verfahren zeichnet sich daher weiterhin dadurch aus, dass bevorzugt Wärme, wie zuvor beschrieben für eine gewisse Zeit von 10 Sekunden bis zu 60 Minuten, besonders von 10 Sekunden bis zu 15 Minuten gemeinsam mit der erfindungsgemäßen Zusammensetzung auf die keratinischen Fasern einwirkt. Dabei kann nach dem Ablauf der Einwirkzeit sowohl die Wärmequelle, also das Handtuch, die Folie zum Abdecken, beispielsweise aus PE oder PP, die Trockenhaube oder der Föhn, entfernt werden und weiterhin die Zusammensetzung wieder ausgespült werden. Erfindungsgemäß umfasst ist jedoch auch die Lehre, dass die erfindungsgemäße Zusammensetzung nach dem Ablauf des erfindungsgemäßen Verfahrens nicht wieder ausgespült wird.

Hinsichtlich der Art, gemäß die erfindungsgemäßen Mittel auf das Haar, aufgebracht werden, bestehen keine prinzipiellen Einschränkungen. Erfindungsgemäß von besonderem Interesse sind Haartonics, insbesondere als auf dem Haar verbleibende (leave on) Formulierung. Diese werden vorzugsweise bei Raumtemperatur angewendet, der alkoholische Gehalt liegt bevorzugt im Bereich von etwa 20 % bis etwa 75 % und der pH-Wert sollte etwa bei pH 4 bis 8 liegen. Insbesondere bei Haartonics hat sich der Einsatz in Liposomen als vorteilhaft erwiesen.

Neben der erfindungsgemäßen zwingend erforderlichen Wirkstoffen können die Mittel prinzipiell alle weiteren, dem Fachmann für solche kosmetischen Mittel bekannten Komponenten enthalten.

Ein ganz besonders bevorzugter weiterer Inhaltsstoff, welcher in besonderer Weise die Wirkung in Bezug auf die Reduktion des Haarbruches deutlich verstärkt, sind Esteröle.

Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Beispiele für die Fettalkoholanteile in den Esterölen sind Isopropylalkohol, Capronalkohol, Caprylalkohol, 2-Ethylhexylalkohol, Caprinalkohol, Laurylalkohol, lsotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®}V).

Selbstverständlich können die Esteröle auch mit Etyhlenoxid, Propylenoxid oder Mischungen aus Ethylenoxid und Propylenoxid alkoxiliert sein. Die Alkoxylierung kann dabei sowohl am Fettalkoholpart als auch am Fettsäurepart als auch an beiden Teilen der Esteröle zu finden sein. Bevorzugt ist erfindungsgemäß jedoch, wenn zunächst der Fettalkohol alkoxyliert wurde und anschließend mit Fettsäure verestert wurde. In der Formel (D4-II) sind allgemein diese Verbindungen dargestellt. 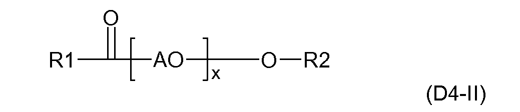 R1 steht hierbei für einen gesättigten oder ungesättigten, verzweigten oder unverzweigten, cyclischen gesättigten cyclischen ungesättigten Acylrest mit 6 bis 30 Kohlenstoffatomen,
AO steht für Ethylenoxid, Propylenoxid oder Butylenoxid,
X steht für eine Zahl zwischen 1 und 200, vorzugsweise 1 und 100, besonders bevorzugt zwischen 1 und 50, ganz besonders bevorzugt zwischen 1 und 20, höchst bevorzugt zwischen 1 und 10 und am bevorzugtesten zwischen 1 und 5,
R2 steht für einen gesättigten oder ungesättigten, verzweigten oder unverzweigten, cyclischen gesättigten cyclischen ungesättigten Alkyl-, Alkenyl-, Alkinyl-, Phenyl- oder Benzylrest mit 6 bis 30 Kohlenstoffatomen. Ein erfindungsgemäß besonders bevorzugtes Esteröl ist beispielsweise unter der INCI - Bezeichnung PPG-3 Benzyl Ether Myristate erhältlich.

Weiterhin sind unter Esterölen zu verstehen:
- Dicarbonsäureester wie Di-n-butyladipat, Di-(2-ethylhexyl)-adipat, Di-(2-ethylhexyl)-succinat und Diisotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat, sowie
- symmetrische, unsymmetrische oder cyclische Ester der Kohlensäure mit Fettalkoholen, beispielsweise Glycerincarbonat oder Dicaprylylcarbonat (Cetiol^{®} CC),
- Trifettsäureester von gesättigten und/oder ungesättigten linearen und/oder verzweigten Fettsäuren mit Glycerin,
- Fettsäurepartialglyceride, das sind Monoglyceride, Diglyceride und deren technische Gemische. Bei der Verwendung technischer Produkte können herstellungsbedingt noch geringe Mengen Triglyceride enthalten sein. Vorzugsweise werden Ölsäuremonoglyceride eingesetzt.

Die Esteröle werden in den erfindungsgemäßen Mitteln in einer Menge von 0,01 bis 20 Gew.%, bevorzugt 0,01 bis 10,0 Gew.%, besonders bevorzugt 0,01 bis 7,5 Gew.%, höchst bevorzugt von 0,1 bis 5,0 Gew.% verwendet.

Erfindungsgemäß bevorzugt enthalten die erfindungsgemäßen Mittel weiterhin mindestens eine quartäre Imidazolinverbindung, d.h. eine Verbindung, die einen positiv geladenen Imidazolinring aufweist. Die im folgenden dargestellte Formel la zeigt die Struktur dieser Verbindungen.

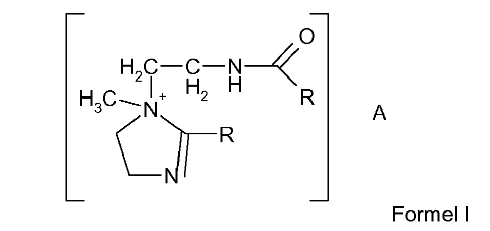

Die Reste R stehen unabhängig voneinander jeweils für einen gesättigten oder ungesättigten, linearen oder verzweigten Kohlenwasserstoffrest mit einer Kettenlänge von 8 bis 30 Kohlenstoffatomen. Die bevorzugten Verbindungen der Formel la enthalten für R jeweils den gleichen Kohlenwasserstoffrest. Die Kettenlänge der Reste R beträgt mindestens 12 Kohlenstoffatome. Bevorzugt sind Verbindungen mit einer Kettenlänge von mindestens 16 Kohlenstoffatomen, besonders bevorzugt mit mindestens 18 Kohlenstoffatomen und höchst bevorzugt mit mindestens 20 Kohlenstoffatomen. A bedeutet ein physiologisch verträgliches Anion. Erfindungsgemäß umfasst sind als anionisches Gegenion Halogenide, beispielsweise Fluorid, Chlorid oder Bromid, Alkylsulfate, wie Methosulfat oder Ethosulfat, Phosphate, Citrat, Tartrat, Maleat oder Fumarat. Diese Anionen stehen erfindungsgemäß in allen Fällen auch für die im folgenden beschriebenen Kationen als deren Gegenion.

Besonders erfindungsgemäße Beispiele sind beispielsweise unter den INCII - Bezeichnungen Quaternium-27, Quaternium-72, Quaternium-83 und Quaternium-91 bekannt

Die Imidazoline der Formel I sind in den erfindungsgemäßen Zusammensetzungen in Mengen von 0,01 bis 20 Gew.%, bevorzugt in Mengen von 0,01 bis 10 Gew.% und ganz besonders bevorzugt in Mengen von 0,1 bis 7,5 Gew.% enthalten. Die allerbesten Ergebnisse werden dabei mit Mengen von 0,1 bis 5 Gew.% jeweils bezogen auf die Gesamtzusammensetzung des jeweiligen Mittels erhalten.

Weiterhin können die folgenden kationischen Tenside gemäß der Formel (Tkat-2) entweder als alleinige kationische Tenside oder ganz besonders bevorzugt als weitere kationische Tenside zusätzlich zu den zuvor genannten Imidazolinen gemäß der Formeln I verwendet werden. RCO-X-N⁺R¹R²R³R⁴ A (Tkat-2) R steht hierin für einen substituierten oder unsubstituierten, verzweigten oder geradkettigen Alkyl- oder Alkenylrest mit 11 bis 35 Kohlenstoffatomen in der Kette,
X steht für - O - oder - NR⁵ -,
R¹ steht für eine Alkylengruppe mit 2 bis 6 C - Atomen, welche nicht substituiert oder substituiert sein kann, wobei im Falle einer Substitution die Substitution mit einer -OH - oder -NH- Gruppe bevorzugt ist, R², R³ und R⁴ jeweils unabhängig voneinander stehen für eine Alkyl oder Hydroxyalkylgruppe mit 1 bis zu 6 C - Atomen in der Kette, wobei die Kette geradlinig oder verzweigt sein kann. Beispiele für erfindungsgemäße Reste sind Methyl, Ethyl, Propyl, iso-Propyl, Butyl, iso-Butyl, tert-Butyl, Pentyl, iso-Pentyl, neo-Pentyl, Hexyl, iso-Hexyl, Hydroxyalkyl, Dihydroxyalkyl, Hydroxyethyl, Hydroxypropyl, Dihydroxypropyl, Hydroxybutyl, Dihydroxybutyl, Trihydroxybutyl, Trihydroxypropyl, Dihydroxyethyl,
R5 steht für Wasserstoff oder einen C1 bis C6 geradkettigen oder verzweigten, Alkyl- oder Alkenylrest, welcher auch durch eine Hydroxygruppe substituiert sein kann, besonders Methyl, Ethyl, Propyl, iso-Propyl, Butyl, iso-Butyl, tert-Butyl, Pentyl, iso-Pentyl, neo-Pentyl, Hexyl, iso-Hexyl, Hydroxyethyl, Hydroxypropyl, Dihydroxypropyl, Hydroxybutyl, Dihydroxybutyl, Trihydroxybutyl, Trihydroxypropyl, Dihydroxyethyl und

Innerhalb dieser Strukturklasse werden bevorzugt die Verbindungen einer der folgenden Strukturen verwendet:

CH₃(CH₂)₂₀CONH(CH₂)₃ - N⁺(CH₃)₂-CH₂CH₃ A⁻ (Tkat-3)

CH₃(CH₂)₂₀CONH(CH₂)₃ - N⁺(CH₃)₂-CH₂(CHOH)CH₂OH A⁻ (Tkat-4)

CH₃(CH2)₂₀COOCH₂CHOHCH₂ - N⁺(CH₃)₃ A⁻ (Tkat-5)

CH₃(CH₂)₂₀CONH(CH₂)₃ - N⁺(CH₃)₂-CH₂CH₂OH A⁻ (Tkat-6)

Beispiele für derartige Handelsprodukte sind Schercoquat BAS, Lexquat AMG-BEO, Akypoquat 131 oder Incroquat Behenyl HE.

Die kationischen Tenside der Formel (Tkat-2) sind in den erfindungsgemäßen Zusammensetzungen in Mengen von 0,01 bis 20 Gew.%, bevorzugt in Mengen von 0,01 bis 10 Gew.% und ganz besonders bevorzugt in Mengen von 0,1 bis 7,5 Gew.% enthalten. Die allerbesten Ergebnisse werden dabei mit Mengen von 0,1 bis 5 Gew.% jeweils bezogen auf die Gesamtzusammensetzung des jeweiligen Mittels erhalten.

Weiterhin können Esterquats gemäß der Formel (Tkat1-2) verwendet werden.

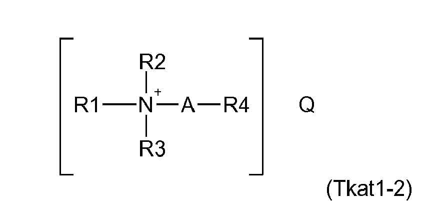

Hierin sind die Reste R1, R2 und R3 jeweils unabhängig voneinander und können gleich oder verschieden sein. Die Reste R1, R2 und R3 bedeuten:
- ein verzweigter oder unverzweigter Alkylrest mit 1 bis 4 Kohlenstoffatomen, welcher mindestens eine Hydroxylgruppe enthalten kann, beispielsweise Methyl, Ethyl, Propyl, Isopropyl, Butyl, Isobutyl, t-Butyl, Hydroxyethyl, Hydroxymethyl, oder
- ein gesättigter oder ungesättigter, verzweigter oder unverzweigter oder ein cyclischer gesättigter oder ungesättigter Alkylrest mit 6 bis 30 Kohlenstoffatomen, welcher mindestens eine Hydroxylgruppe enthalten kann, oder
- ein Aryl oder Alkarylrest, beispielsweise Phenyl oder Benzyl,
- den Rest (- A - R4), mit der Maßgabe, daß höchstens 2 der Reste R1, R2 oder R3 für diesen Rest stehen können:

Der Rest -(A - R4) ist mindestens 1 bis 3 mal enthalten.

Hierin steht A für:
1) -(CH2)n- mit n = 1 bis 20, vorzugsweise n = 1 bis 10 und besonders bevorzugt n = 1 - 5, oder
2) -(CH2-CHR5-O)n- mit n = 1 bis 200, vorzugsweise 1 bis 100, besonders bevorzugt 1 bis 50, und besonders bevorzugt 1 bis 20 mit R5 in der Bedeutung von Wasserstoff, Methyl oder Ethyl,
und R4 steht für:
1) R6-O-CO-, worin R6 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann, oder
2) R7-CO-, worin R7 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Rewoquat^{®}, Stepantex^{®}, Dehyquart^{®} und Armocare^{®} vertrieben. Die Produkte Armocare^{®} VGH-70, ein N,N-Bis(2-Palmitoyloxyethyl)dimethylammonium-chlorid, sowie Dehyquart^{®} F-75, Dehyquart^{®} C-4046, Dehyquart^{®} L80, Dehyquart^{®} F-30, Dehyquart^{®} AU-35, Rewoquat^{®} WE18, Rewoquat^{®} WE38 DPG und Stepantex^{®} GS 90 sind Beispiele für solche Esterquats.

Weitere erfindungsgemäß besonders bevorzugte Verbindungen der Formel (Tkat1-2) zählen zur Formel (Tkat1-2.1), den kationischen Betainestern.

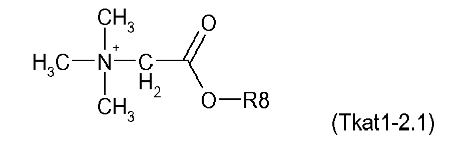

R8 entspricht in seiner Bedeutung R7.

Die Esterquats sind in den erfindungsgemäßen Zusammensetzungen in Mengen von 0,01 bis 20 Gew.%, bevorzugt in Mengen von 0,01 bis 10 Gew.% und ganz besonders bevorzugt in Mengen von 0,1 bis 7,5 Gew.% enthalten. Die allerbesten Ergebnisse werden dabei mit Mengen von 0,1 bis 5 Gew.% jeweils bezogen auf die Gesamtzusammensetzung des jeweiligen Mittels erhalten.

Als weiterer Inhaltsstoff können Monoalkyltrimethylammoniumsalze mit einer Kettenlänge des Alkylrestes von 16 bis 24 Kohlenstoffatomen enthalten sein.

Diese Verbindungen weisen die in der Formel (Tkat1-1) dargestellte Struktur auf,

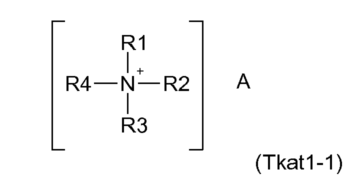

Wobei R1, R2 und R3 für jeweils eine Methylgruppe stehen und R4 für einen gesättigten, verzweigten oder unverzweigten Alkylrest mit einer Kettenlänge von 16 bis 24 Kohlenstoffatomen.

Besonders bevorzugte Verbindungen der Formel (Tkat1-1) haben als Anion das Chlorid oder Methosulfat mit einer Methylgruppe als Rest R, und weiterhin als Rest R4 einen gesättigten, verzweigten oder unverzweigten Alkylrest, ganz besonders bevorzugt unverzweigten Alkylrest mit einer Kettenlänge von 18 bis 24, höchst bevorzugt mit einer Kettenlänge von 22 bis 24 Kohlenstoffatomen.

Beispiele für Verbindungen der Formel (Tkat1-1) sind Cetyltrimethylammoniumchlorid, Cetyltrimethylammoniumbromid, Cetyltrimethylammoniummethosulfat, Stearyltrimethylammoniumchlorid, Behenyltrimethylammoniumchlorid, Behenyltrimethylammoniumbromid und Behenyltrimethylammoniummethosulfat.

Die Verbindungen der Formel (Tkat1-1) werden in den erfindungsgemäßen Zusammensetzungen in einer Menge von 0,01 bis 5,0 Gew.% verwendet. Bevorzugt werden 0,1 bis 5,0 Gew.% verwendet. Besonders bevorzugt sind Mengen von 0,1 bis 3,0 Gew.%. Die Mengenangaben beziehen sich jeweils auf die gesamte Zusammensetzung.

Weiterhin enthalten die erfindungsgemäßen Mittel bevorzugt mindestens ein Amin und/oder kationisiertes Amin, insbesondere ein Amidoamin und/oder ein kationisiertes Amidoamin mit den folgenden Strukturformeln:

R¹ - NH - (CH₂)ₙ - NR²R³ (Tkat7)

und/oder

R¹ - NH - (CH₂)ₙ - NR²R³R⁴ (Tkat8)

worin R1 ein Acyl-oder Alkylrest mit 6 bis 30 C-Atomen, welche verzweigt oder unverzweigt, gesättigt oder ungesättigt sein können, und wobei der Acylrest und/oder der Alkylrest mindestens eine OH-Gruppe enthalten können, und
R2, R3 und R4 jeweils unabhängig voneinander Wasserstoff oder ein Alkylrest mit 1 bis 4 C-Atomen, welcher gleich oder verschieden, gesättigt oder ungesättigt sein kann, und
X ein Anion und
n eine ganze Zahl zwischen 1 und 10 bedeuten.

Das Anion ist ausgewählt aus den physiologisch verträglichen Anionen. Beispielhaft hierfür seien die Halogenidionen, Fluorid, Chlorid, Bromid, Sulfat der allgemeinen Formel RSO₃, worin R die Bedeutung von gesättigtem oder ungesättigtem Alkylresten mit 1 bis 4 Kohlenstoffatomen hat, oder anionische Reste organischer Säuren wie Maleat, Fumarat, Oxalat, Tartrat, Citrat, Lactat oder Acetat, genannt.

Bevorzugt wird eine Zusammensetzung, in welcher das Amin und/oder das quaternisierte Amin gemäß allgemeiner Formeln (Tkat7) und/oder (Tkat8) ein Amidoamin und/oder ein quaternisiertes Amidoamin ist, worin R1 ein verzweigter oder unverzweigter, gesättigter oder ungesättigter Acylrest mit 6 bis 30 C-Atomen, welcher mindestens eine OH-Gruppe enthalten kann, bedeutet. Bevorzugt ist hierbei ein Fettsäurerest aus Ölen und Wachsen, insbesondere aus natürlichen Ölen und Wachsen, ist. Als Beispiele hierfür kommen Lanolin, Bienen-oder Candellilawachse in Betracht.

Bevorzugt sind auch solche Amidoamine und/oder quaternisierte Amidoamine, in denen R2, R3 und/oder R4 in Formeln (Tkat7) und/oder (Tkat8) ein Rest gemäß der allgemeinen Formel CH₂CH₂OR5 bedeuten, worin R5 die Bedeutung von Alkylresten mit 1 bis 4 Kohlenstoffatomen, Hydroxyethyl oder Wasserstoff haben kann. Die bevorzugte Größe von n in den allgemeinen Formeln (Tkat7) und/oder (Tkat8) ist eine ganze Zahl zwischen 2 und 5.

Weiterhin bevorzugt sind Amidoamine und/oder quaternisierte Amidoamine der allgemeinen Formeln (Tkat7) und/oder (Tkat8), in denen das Anion X ein Halogenidion oder eine Verbindung der allgemeinen Formel RSO₃⁻ ist, worin R die Bedeutung von gesättigtem oder ungesättigtem Alkylresten mit 1 bis 4 Kohlenstoffatomen hat.

Der Alkylrest mit 1 bis 4 Kohlenstoffatomen von R2, R3 und R4 und/oder der Alkylrest mit 1 bis 4 Kohlenstoffatomen von RSO₃⁻ in der allgemeinen Formel (Tkat7) und/oder (Tkat8) können mindestens eine Hydroxylgruppe enthalten.

Die Alkylamidoamine können sowohl als solche vorliegen und durch Protonierung in entsprechend saurer Lösung in eine quaternäre Verbindung in der Zusammensetzung überführt werden. Erfindungsgemäß bevorzugt sind die kationischen Alkylamidoamine.

Als erfindungsgemäß zu verwendende Amidoamine, welche gegebenenfalls quaternisiert sein können, kommen beispielsweise in Betracht als Amidoamine: Witcamine 100 (Witco, INCI-Bezeichnung: Cocamidopropyl Dimethylamine), Incromine BB (Croda, INCI-Bezeichnung: Behenamidopropyl Dimethylamine), Mackine 401 (Mclntyre, INCI-Bezeichnung: Isostearylamidopropyl Dimethylamine) und andere Mackine-Typen, Adogen S18V (Witco, INCI-Bezeichnung: Stearylamidopropyl Dimethylamine), und als permanent kationische Aminoamine: Rewoquat RTM 50 (Witco Surfactants GmbH, INCI-Bezeichnung: Ricinoleamidopropyltrimonium Methosulfate), Empigen CSC (Albright&Wilson, INCI-Bezeichnung: Cocamidopropyltrimonium Chlorid), Swanol Lanoquat DES-50 (Nikko, INCI-Bezeichnung: Quatemium-33), Rewoquat UTM 50 (Witco Surfactants GmbH, Undecyleneamidopropyltrimonium Methosulfate).

Die Amidoamine oder quaternisierten Amidoamine gemäß der allgemeinen Formeln (Tkat7) und (Tkat8) können einzeln oder in beliebigen Kombinationen miteinander verwendet werden, wobei Mengen zwischen 0,01 bis 20 Gew.%, bevorzugt in Mengen von 0,01 bis 10 Gew.% und ganz besonders bevorzugt in Mengen von 0,1 bis 7,5 Gew.% enthalten. Die allerbesten Ergebnisse werden dabei mit Mengen von 0,1 bis 5 Gew.% jeweils bezogen auf die Gesamtzusammensetzung des jeweiligen Mittels erhalten.

Als besonders bevorzugter Inhaltsstoff ist mindestens eine kationisch geladene polymere Verbindung enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Die kationischen Polymere können Homo- oder Copolymere sein, wobei die quaternären Stickstoffgruppen entweder in der Polymerkette oder vorzugsweise als Substituent an einem oder mehreren der Monomeren enthalten sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie zum Beispiel Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen. Die Ammoniumgruppen enthaltenden Monomere können mit nicht kationischen Monomeren copolymerisiert sein. Geeignete Comonomere sind beispielsweise Acrylamid, Methacrylamid; Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignete Polymere mit quaternären Amingruppen sind beispielsweise die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16) oder quaternisiertes Vinylpyrrolidon/Dimethylaminoethylmethacrylat Copolymer (Polyquaternium- 11).

Von den kationischen Polymeren, die in dem erfindungsgemäßen Mittel enthalten sein können, ist zum Beispiel Vinylpyrrolidon/Dimethylaminoethylmethacrylatmethosulfat Copolymer, das unter den Handelsbezeichnungen Gafquat^{®} 755 N und Gafquat^{®} 734 von der Firma Gaf Co., USA vertrieben wird und von denen das Gafquat^{®} 734 besonders bevorzugt ist, geeignet. Weitere kationische Polymere sind beispielsweise das von der Firma BASF, Deutschland unter dem Handelsnamen Luviquat^{®} HM 550 vertriebene Copolymer aus Polyvinylpyrrolidon und lmidazoliminmethochlorid, das von der Firma Calgon/USA unter dem Handelsnamen Merquat^{®} Plus 3300 vertriebene Terpolymer aus Dimethyldiallylammoniumchlorid, Natriumacrylat und Acrylamid und das von der Firma ISP unter dem Handelsnamen Gafquat^{®} HS 100 vertriebene Vinylpyrrolidon/Methacrylamidopropyltrimethylammoniumchlorid Copolymer.

### Homopolymere der allgemeinen Formel (P1),

-{CH₂-[CR¹COO-(CH₂)ₘN⁺R²R³R⁴]}ₙ X⁻ (P1)

in der R¹= -H oder -CH₃ ist,
R², R³ und R⁴ unabhängig voneinander ausgewählt sind aus C1-4-Alkyl-, -Alkenyl- oder - Hydroxyalkylgruppen,
m = 1, 2, 3 oder 4,
n eine natürliche Zahl und
X⁻ ein physiologisch verträgliches organisches oder anorganisches Anion ist, sowie Copolymere, bestehend im wesentlichen aus den in Formel (Monomer-3) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹ steht für eine Methylgruppe
- R², R³ und R⁴ stehen für Methylgruppen
- m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Solche Produkte sind beispielsweise unter den Bezeichnungen Rheocare® CTH (Cosmetic Rheologies) und Synthalen® CR (3V Sigma) im Handel erhältlich.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 und Salcare^{®} SC 96 im Handel erhältlich.

Copolymere enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Geeignete kationische Polymere, die von natürlichen Polymeren abgeleitet sind, sind kationische Derivate von Polysacchariden, beispielsweise kationische Derivate von Cellulose, Stärke oder Guar. Geeignet sind weiterhin Chitosan und Chitosanderivate. Kationische Polysaccharide haben die allgemeine Formel (P-3) G-O-B-N+RₐR_{b}R_{c} X⁻

G ist ein Anhydroglucoserest, beispielsweise Stärke- oder Celluloseanhydroglucose;

B ist eine divalente Verbindungsgruppe, beispielsweise Alkylen, Oxyalkylen, Polyoxyalkylen oder Hydroxyalkylen;

Rₐ, R_{b} und R_{c} sind unabhängig voneinander Alkyl, Aryl, Alkylaryl, Arylalkyl, Alkoxyalkyl oder Alkoxyaryl mit jeweils bis zu 18 C-Atomen, wobei die Gesamtzahl der C-Atome in Rₐ, R_{b} und R_{c} vorzugsweise maximal 20 ist;

X⁻ ist ein übliches Gegenanion und ist vorzugsweise Chlorid.

Eine kationische Cellulose wird unter der Bezeichnung Polymer JR^{®} 400 von Amerchol vertrieben und hat die INCI-Bezeichnung Polyquaternium-10. Eine weitere kationische Cellulose trägt die INCI-Bezeichnung Polyquaternium-24 und wird unter dem Handelsnamen Polymer LM-200 von Amerchol vertrieben. Weitere Handelsprodukte sind die Verbindungen Celquat^{®} H 100, Celquat^{®} und L 200. Die genannten Handelsprodukte sind bevorzugte kationische Cellulosen.

Geeignete kationische Guarderivate werden unter der Handelsbezeichnung Jaguar^{®} vertrieben und haben die INCI-Bezeichnung Guar Hydroxypropyltrimonium Chloride. Weiterhin werden besonders geeignete kationische Guarderivate auch von der Fa. Hercules unter der Bezeichnung N-Hance^{®} im Handel. Weitere kationische Guarderivate werden von der Fa. Cognis unter der Bezeichnung Cosmedia® vertrieben. Ein bevorzugtes kationisches Guarderivat ist das Handelsprodukt AquaCat^{®} der Fa. Hercules. Bei diesem Rohstoff handelt es sich um ein bereits vorgelöstes kationisches Guarderivat.

Ein weiteres besonders geeignetes kationisches natürliches Polymer stellen Hydrokolloide von Typ der Chitosane dar. Die Chitosane oder Chitosanderivate liegen vorzugsweise in neutralisierter oder partiell neutralisierter Form vor. Ein geeignetes Chitosan wird beispielsweise von der Firma Kyowa Oil& Fat, Japan, unter dem Handelsnamen Flonac^{®} vertrieben. Ein bevorzugtes Chitosansalz ist Chitosoniumpyrrolidoncarboxylat, welches beispielsweise unter der Bezeichnung Kytamer^{®} PC von der Firma Amerchol, USA, vertrieben wird. Weitere Chitosanderivate sind unter den Handelsbezeichnungen Hydagen^{®} CMF, Hydagen^{®} HCMF und Chitolam^{®} NB/101 im Handel frei verfügbar.

Weitere bevorzugte kationische Polymere sind beispielsweise
- kationische Alkylpolyglycoside,
- kationisierter Honig, beispielsweise das Handelsprodukt Honeyquat^{®} 50,
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette,
- Vinylpyrrolidon-Vinylcaprolactam-Acrylat-Terpolymere, wie sie mit Acrylsäureestern und Acrylsäureamiden als dritter Monomerbaustein im Handel beispielsweise unter der Bezeichnung Aquaftex^{®} SF 40 angeboten werden.

Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weiterhin sind kationisierte Proteinhydrolysate zu den kationischen Polymeren zu zählen. Das zugrunde liegende Proteinhydrolysat kann vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen.

Ganz besonders bevorzugt sind die kationischen Proteinhydrolysate und -derivate auf pflanzlicher Basis. Die kationischen Polymere sind in den erfindungsgemäßen Zusammensetzungen bevorzugt in Mengen von 0,01 bis 10 Gew.-%, bezogen auf das gesamte Mittel, enthalten. Mengen von 0,05 bis 5 Gew.-% sind besonders bevorzugt.

Amphotere Polymere sind ebenso wie die kationischen Polymere ganz besonders bevorzugte Polymere. Erfindungsgemäß bevorzugte amphotere und/oder kationische Polymere sind solche Polymerisate, in denen sich eine kationische Gruppe ableitet von mindestens einem der folgenden Monomere:
- Monomeren mit quartären Ammoniumgruppen der allgemeinen Formel (Mono1),

   R¹-CH=CR²-CO-Z-(CₙH₂ₙ)-N⁽⁺⁾R³R⁴R⁵ A⁽⁻⁾ (Mono1)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist,
- Monomeren mit quartären Ammoniumgruppen der allgemeinen Formel (Mono2), 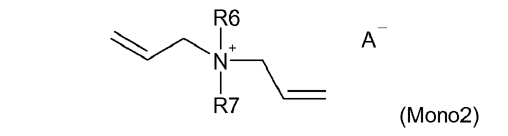 worin R⁶ und R⁷ unabhängig voneinander stehen für eine (C₁ bis C₄)-Alkylgruppe, insbesondere für eine Methylgruppe und
   A⁻ das Anion einer organischen oder anorganischen Säure ist.

Wenn sich eine kationische Gruppe der amphoteren bzw. kationischen Polymerisate vom Monomer der Formel (Mono1) ableitet, stehen die Reste R³, R⁴ und R⁵ in Formel (Mono1) bevorzugt für Methylgruppen, Z ist bevorzugt eine NH-Gruppe und A⁽⁻⁾ bedeutet bevorzugt ein Halogenid-, Methoxysulfat- oder Ethoxysulfat-lon. Besonders bevorzugt ist es in diesem Falle Acrylamidopropyl-trimethylammoniumchlorid als Monomer (Mono1) zu verwenden.

In Formel (Mono2) steht A⁻ bevorzugt für ein Halogenidion, insbesondere für Chlorid oder Bromid. Bevorzugte erfindungsgemäße amphotere Polymere sind Polymere, deren anionische Gruppe sich von mindestens einem Monomeren der Formel (Mono3) ableitet
- monomeren Carbonsäuren der allgemeinen Formel (Mono3) bzw. deren Salze mit einer organischen oder anorganischen Säure,

   R⁸-CH=CR⁹-COOH (Mono3)

   in denen R⁸ und R⁹ unabhängig voneinander Wasserstoff oder Methylgruppen sind.

Als Monomeres (Mono3) wird für die erfindungsgemäß bevorzugten amphoteren Polymerisate Acrylsäure verwendet.

Besonders bevorzugte amphotere Polymere sind Copolymere, aus mindestens einem Monomer (Mono1) bzw. (Mono2) mit dem Momomer (Mono3), insbesondere Copolymere aus den Monomeren (Mono2) und (Mono3). Erfindungsgemäß ganz besonders bevorzugt verwendete amphotere Polymere sind Copolymerisate aus Diallyl-dimethylammoniumchlorid und Acrylsäure. Diese Copolymerisate werden unter der INCI-Bezeichnung Polyquaternium-22 unter anderem mit dem Handelsnamen Merquat^{®} 280 (Nalco) vertrieben.

Darüber hinaus können die erfindungsgemäßen amphoteren Polymere neben einem Monomer Mono1) oder (Mono2) und einem Monomer (Mono3) zusätzlich ein Monomer (Mono4)
- monomere Carbonsäureamide der allgemeinen Formel (Mono4), 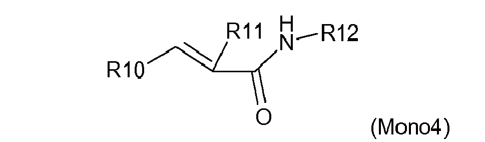 in denen R¹⁰ und R¹¹ unabhängig voneinander Wasserstoff oder Methylgruppen sind und R¹² für ein Wasserstoffatom oder eine (C₁- bis C₈)-Alkylgruppe steht, enthalten.

Erfindungsgemäß ganz besonders bevorzugt verwendete amphotere Polymere auf Basis eines Comonomers (Mono4) sind Terpolymere aus Diallyldimethylammoniumchlorid, Acrylamid und Acrylsäure. Diese Copolymerisate werden unter der INCI-Bezeichnung Polyquaternium-39 unter anderem mit dem Handelsnamen Merquat^{®} Plus 3330 (Nalco) vertrieben.

Die amphoteren Polymere sind in den erfindungsgemäßen Mitteln bevorzugt in Mengen von 0,01 bis 10 Gew.-%, bezogen auf das gesamte Mittel, enthalten. Mengen von 0,01 bis 5 Gew.-% sind besonders bevorzugt.

Als weiteren Inhaltstoff zusätzlich zu den zwingenden Komponenten enthalten die erfindungsgemäßen Mittel bevorzugt mindestens ein Silikonpolymer ausgewählt aus der Gruppe der Dimethiconole und/oder der Gruppe der aminofunktionellen Silikone und / oder der Gruppe der Dimethicone und / oder der Gruppe der Cyclomethicone. Diese Inhaltsstoffe werden im folgenden beschrieben.

Die erfindungsgemäßen Dimethicone können sowohl linear als auch verzweigt als auch cyclisch oder cyclisch und verzweigt sein. Lineare Dimethicone können durch die folgende Strukturformel (Si1) dargestellt werden:

(SiR¹₃) - O - (SiR²₂ - O - )ₓ - (SiR¹₃) (Si1)

Verzweigte Dimethicone können durch die Strukturformel (Si1.1) dargestellt werden:

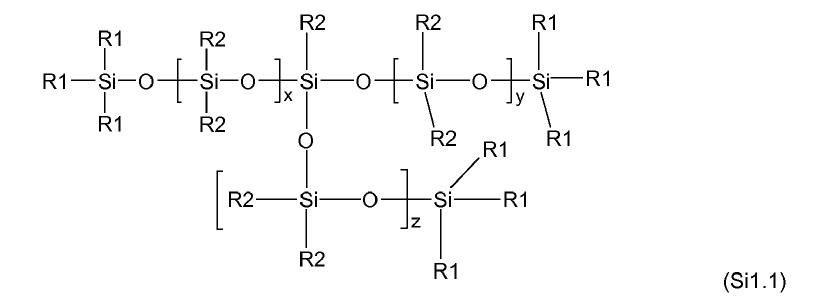

Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Die Zahlen x, y und z sind ganze Zahlen und laufen jeweils unabhängig voneinander von 0 bis 50.000. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Höchst bevorzugt sind Viskositäten um den Bereich von etwa 60.000 cPs herum. Beispielhaft sei hier auf das Produkt "Dow Corning 200 mit 60000cSt" verwiesen.

Die Dimethicone (Si1) sind in den erfindungsgemäßen Zusammensetzungen in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% bezogen auf die gesamte Zusammensetzung enthalten.

Besonders bevorzugte erfindungsgemäße Mittel enthalten ein oder mehrere aminofunktionelle Silikone.

Erfindungsgemäß geeignet sind kationische Silikonöle wie beispielsweise die im Handel erhältliche Dow Corning 929 Emulsion, DC 2-2078 (Hersteller Dow Corning, INCI-Bezeichnung: Aminopropyl Phenyl Trimethicone), DC 5-7113 (Hersteller Dow Corning, INCI-Bezeichnung: Silicone Quaternium 16), SM-2059 (Hersteller: General Electric) sowie SLM-55067 (Hersteller: Wacker).

Besonders bevorzugte erfindungsgemäße Mittel sind **dadurch gekennzeichnet, dass** sie mindestens es ein aminofunktionelles Silikon der Formel (Si3-a) 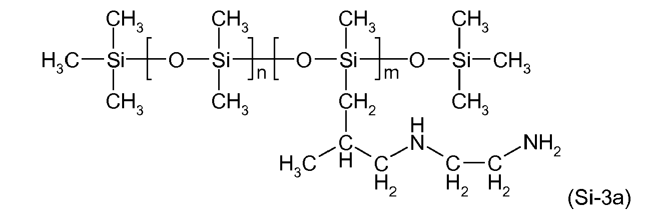 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Trimethylsilylamodimethicone bezeichnet und sind beispielsweise unter der Bezeichnung Q2-7224 (Hersteller: Dow Corning; ein stabilisiertes Trimethylsilylamodimethicon) erhältlich.

Besonders bevorzugt sind auch erfindungsgemäße Mittel, die mindestens ein aminofunktionelles Silikon der Formel (Si-3b) 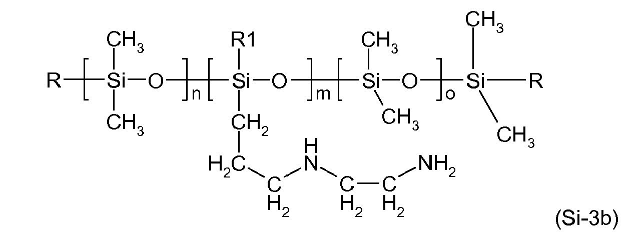 enthalten, worin
- R: für -OH, eine (gegebenenfalls ethoxylierte und/oder propoxylierte) (C₁ bis C₂₀)- Alkoxygruppe oder eine -CH₃-Gruppe steht,
- R': für -OH, eine (C₁ bis C₂₀)-Alkoxygruppe oder eine -CH₃-Gruppe und
- m, n1 und n2: Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Amodimethicone, bzw. als funktionalisierte Amodimethicone, wie beispielsweise Bis(C13-15 Alkoxy) PG Amodimethicone (beispielsweise als Handelsprodukt: DC 8500 der Firma Dow Corning erhältlich), Trideceth-9 PG-Amodimethicone (beispielsweise als Handelsprodukt Silcare Silicone SEA der Firma Clariant erhältlich) bezeichnet. Neben den zuvor beschriebenen aminofunktionellen Silikonen können bevorzugt aminofunktionelle Silikone verwendet werden, welche sich durch endständige Aminogruppen, welche wiederum besonders bevorzugt quaternär sind. Hervorragend geeignet sind hierbei diquaternäre Silikone. Geeignete diquaternäre Silikone sind ausgewählt aus Verbindungen der allgemeinen Formel (Si3c)

[R¹R²R³N⁺ - A - SiR⁷R⁸ - (O-SiR⁹R¹⁰)ₙ - O - SiR¹¹R¹² - A - N⁺R⁴R⁵R⁶] 2X⁻ (Si3c)

wobei die Reste R1 bis R6 unabhängig voneinander C1-bis C22-Alkylreste bedeuten, welche Hydroxygruppen enthalten können und wobei vorzugsweise mindestens einer der Reste mindestens 8 C-Atome aufweist und die übrigen Reste 1 bis 4 C-Atome aufweisen,
die Reste R7 bis R12 unabhängig voneinander gleich oder verschieden sind und C1-bis C10-Alkyl oder Phenyl bedeuten,
A eine divalente organische Verbindungsgruppe bedeutet,
n eine Zahl von 0 bis 200, vorzugsweise von 10 bis 120, besonders bevorzugt von 10 bis 40 ist, und
X⁻ ein Anion ist.

Die divalente Verbindungsgruppe ist vorzugsweise eine C1-bis C12-Alkylen-oder Alkoxyalkylengruppe, die mit einer oder mehreren Hydroxylgruppen substituiert sein kann. Besonders bevorzugt ist die Gruppe -(CH₂)₃-O-CH₂-CH(OH)-CH₂-.

Das Anion X⁻ kann ein Halogenidion, ein Acetat, ein organisches Carboxylat oder eine Verbindung der allgemeinen Formel RSO₃⁻ sein, worin R die Bedeutung von C1-bis C4-Alkylresten hat.

Ein bevorzugtes diquaternäres Silikon hat die allgemeine Formel (Si3d)

[RN⁺Me₂-A-(SiMe₂O)ₙ-SiMe₂-A-N⁺Me₂R] 2CH₃COO⁻ (Si3d),

wobei A die Gruppe -(CH₂)₃-O- CH₂- CH(OH) - CH₂-ist,

R ein Alkylrest mit mindestens 8 C-Atomen und n eine Zahl von 10 bis 120 ist.

Geeignete Silikonpolymere mit zwei endständigen, quaternären Ammoniumgruppen sind unter der INCI-Bezeichnung Quaternium-80 bekannt. Hierbei handelt es sich um Dimethylsiloxane mit zwei endständigen Trialkylammoniumgruppen. Derartige diquaternäre Polydimethylsiloxane werden von der Firma Evonik unter den Handelsnamen Abil^{®} Quat 3270, 3272 und 3474 vertrieben.

Erfindungsgemäß bevorzugte kosmetische Zubereitungen sind **dadurch gekennzeichnet, dass** sie, bezogen auf ihr Gewicht, 0,01 bis 10 Gew.%, vorzugsweise 0,01 bis 8 Gew.%, besonders bevorzugt 0,1 bis 7,5 Gew.% und insbesondere 0,2 bis 5 Gew.% aminofunktionelle(s) Silikon(e) und/oder diquaternäres Silikon enthalten.

Auch die nach INCI als Cyclomethicone bezeichneten cyclischen Dimethicone sind erfindungsgemäß mit Vorzug einsetzbar. Hier sind erfindungsgemäße kosmetische oder dermatologische Zubereitungen bevorzugt, die mindestens ein Silikon der Formel (Si-4) 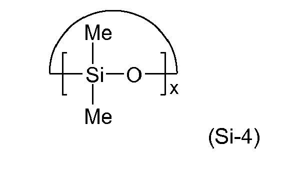 enthalten, in der x für eine Zahl von 3 bis 200, vorzugsweise von 3 bis 10, weiter bevorzugt von 3 bis 7 und insbesondere 3, 4, 5 oder 6, steht.

Erfindungsgemäß ebenfalls bevorzugte Mittel sind **dadurch gekennzeichnet, dass** sie mindestens ein Silikon der Formel (Si-5)

R₃Si-[O-SiR₂]ₓ-(CH₂)ₙ-[O-SiR₂]y-O-SiR₃ (Si-5),

enthalten, in der R für gleiche oder verschiedene Reste aus der Gruppe -H, -Phenyl, -Benzyl, -CH₂-CH(CH₃)Ph, der C₁₋₂₀-Alkylreste steht, x bzw. y für eine Zahl von 0 bis 200 steht.

Als weitere Silikone neben den erfindungsgemäßen Dimethiconen, Dimethiconolen, Amodimethiconen und/oder Cyclomethiconen können wasserlösliche Silikone in den erfindungsgemäßen Zusammensetzungen enthalten sein.

Entsprechende hydrophile Silikone werden beispielsweise aus den Verbindungen der Formeln (Si-6) und/oder (Si-7) ausgewählt. Insbesondere bevorzugte wasserlösliche Tenside auf Silikonbasis sind ausgewählt aus der Gruppe der Dimethiconcopolyole die bevorzugt alkoxyliert, insbesondere polyethoxyliert oder polypropoxyliert sind.

Unter Dimethiconcopolyolen werden erfindungsgemäß bevorzugt Polyoxyalkylen-modifizierte Dimethylpolysiloxane der allgemeinen Formeln (Si-6) oder (Si-7) verstanden: 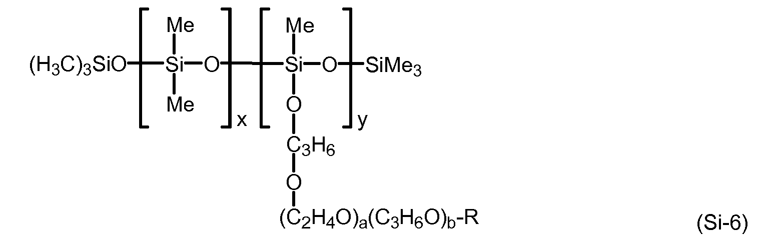 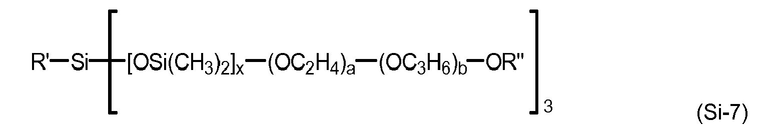 worin
- der Rest R steht für ein Wasserstoffatom, eine Alkylgruppe mit 1 bis 12 C-Atomen, eine Alkoxygruppe mit 1 bis 12 C-Atomen oder eine Hydroxylgruppe,
- die Reste R' und R" bedeuten Alkylgruppen mit 1 bis 12 C-Atomen,
- x steht für eine ganze Zahl von 1 bis 100, bevorzugt von 20 bis 30,
- y steht für eine ganze Zahl von 1 bis 20, bevorzugt von 2 bis 10 und
- a und b stehen für ganze Zahlen von 0 bis 50, bevorzugt von 10 bis 30.

Besonders bevorzugte Dimethiconcopolyole im Sinne der Erfindung sind beispielsweise die kommerziell unter dem Handelsnamen SILWET (Union Carbide Corporation) und DOW CORNING (Dow) vertriebenen Produkte.

Erfindungsgemäß besonders bevorzugte Dimethiconcopolyole sind Dow Corning 190 und Dow Corning 193 (Dow).

Die Dimethiconcopolyole sind in den erfindungsgemäßen Zusammensetzungen in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% an Dimethiconcopolyol bezogen auf die Zusammensetzung.

Schließlich werden unter den Silikonverbindungen die Dimethiconole (Si8) verstanden. Dimethiconole bilden eine weitere Gruppe der Silikone, welche erfindungsgemäß besonders bevorzugt sind. Die erfindungsgemäßen Dimethiconole können sowohl linear als auch verzweigt als auch cyclisch oder cyclisch und verzweigt sein. Lineare Dimethiconole können durch die folgende Strukturformel (Si8 - I) dargestellt werden:

(SiOHR¹₂) - O - (SiR²₂ - O -)ₓ - (SiOHR¹₂) (Si8 - I)

Verzweigte Dimethiconole können durch die Strukturformel (Si8 - II) dargestellt werden:

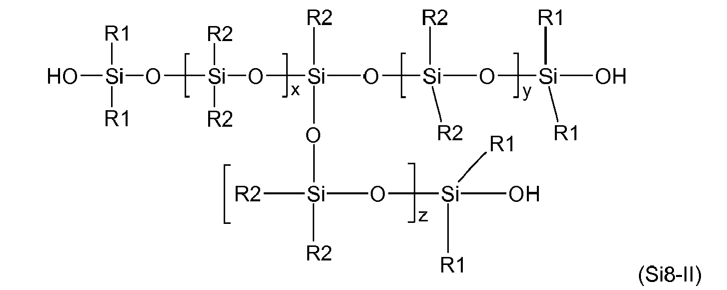

Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Die Zahlen x, y und z sind ganze Zahlen und laufen jeweils unabhängig voneinander von 0 bis 50.000. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Der bevorzugteste Bereich liegt zwischen 50000 und 2000000 cPs.

Als Beispiele für derartige Produkte werden die folgenden Handelsprodukte genannt: Dow Corning 1-1254 Fluid, Dow Corning 2-9023 Fluid, Dow Corning 2-9026 Fluid, Abil OSW 5 (Degussa Care Specialties), Dow Corning 1401 Fluid, Dow Corning 1403 Fluid, Dow Corning 1501 Fluid, Dow Corning 1784 HVF Emulsion, Dow Corning 9546 Silicone Elastomer Blend (alle zuvor genannten Dow Corning Corporation), SM555, SM2725, SM2765, SM2785 (alle zuvor genannten GE Silicones), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH).

Die Dimethiconole (Si8) sind in den erfindungsgemäßen Zusammensetzungen in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% an Dimethiconol bezogen auf die Zusammensetzung.

Weiterhin können zusätzlich mit der erfindungsgemäßen Wirkstoffkombination (A) kosmetische Öle verwendet werden. Bevorzugt weisen diese Ölkörper einen Schmelzpunkt kleiner als 50 °C, besonders bevorzugt kleiner als 45 °C, ganz besonders bevorzugt kleiner als 40 °C, höchst bevorzugt kleiner als 35 °C und am bevorzugtesten sind die kosmetischen Öle bei einer Temperatur kleiner als 30 °C fließfähig. Im folgenden werden diese Öle näher definiert und beschrieben.

Zu den natürlichen und synthetischen kosmetischen Ölen sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Din-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.

Als natürliche Öle kommen beispielsweise Amaranthsamenöl, Aprikosenkernöl, Arganöl, Avocadoöl, Babassuöl, Baumwollsaatöl, Borretschsamenöl, Camelinaöl, Distelöl, Erdnußöl, Granatapfelkernöl, Grapefruitsamenöl, Hanföl, Haselnussöl, Holundersamenöl, Johannesbeersamenöl, Jojobaöl, Kakaobutter, Leinöl, Macadamianussöl, Maiskeimöl, Mandelöl, Marulaöl, Nachtkerzenöl, Olivenöl, Palmöl, Rapsöl, Reisöl, Sanddornfruchtfleischöl, Sanddornkernöl, Sesamöl, Sheabutter, Sojaöl, Sonnenblumenöl, Traubenkernöl, Walnußöl oder Wildrosenöl.

In vielen Fällen enthalten die Mittel mindestens eine oberflächenaktive Substanz.

Als anionische Tenside (Tanion) eignen sich in erfindungsgemäßen Zubereitungen:
- lineare und verzweigte Fettsäuren mit 8 bis 30 C-Atomen (Seifen),
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate,
- Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- sulfatierte Fettsäurealkylenglykolester,
- Acylglutamate,
- Kondensationsprodukte aus einem wasserlöslichen Salz eines wasserlöslichen Eiweißhydrolysates mit einer C8 - C30 Fettsäure, bevorzugt von Fettsäuren mit 12 - 18 C-Atomen. Solche Produkte sind unter dem Warenzeichen Lamepon^{®}, Maypon^{®}, Gluadin^{®}, Hostapon^{®} KCG oder Amisoft^{®} seit langem im Handel erhältlich,
- Acyllactylate,
- Hydroxymischethersulfate,
- Ethercarbonsäuren,
- Acylsarcoside,
- Acyltauride,
- Acylisethionate,
- Sulfobernsteinsäuremono- und -dialkylester,
- Alkylpolyglykolethersulfate,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate,
- Monoglyceridsulfate und Monoglyceridethersulfate,
- Amidethercarbonsäuren. Derartige Produkte sind beispielsweise von der Firma Chem-Y unter der Produktbezeichnung Akypo^{®} erhältlich.
- Acylaspartate.

Selbstverständlich können alle anionischen Tenside auch in Form ihrer Salze verwendet werden. Besonders geeignete anionische Tenside liegen jeweils in Form der Lithium-, Magnesium-, Zink-, Natrium-, Kalium- und Ammonium- sowie der Mono-, Di- und Trialkanolammoniumsalze mit 1 bis 4 C-Atomen in der Alkanolgruppe vor. Weitere anionische Tenside, welche ganz besonders bevorzugt in der erfindungsgemäßen Zusammensetzung verwendet werden, sind Alkyl- und/oder Alkenyl-Oligoglykosidcarboxylate, -sulfate, -phosphate und/oder -isethionate, die sich von Alkyl- und/oder Alkenyloligoglykosiden ableiten. Speziell bevorzugt ist ein Laurylglucosidcarboxylat, wie es als Plantapon^{®} LCG von Cognis Deutschland erhältlich ist.

Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Typische Beispiele für amphotere bzw. zwitterionische Tenside sind Alkylbetaine, Alkylamidobetaine, Amino-propionate, Aminoglycinate, lmidazoliniumbetaine und Sulfobetaine. Nichtionische Tenside (Tnio) enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 6 bis 30 C-Atomen, die Fettalkoholpolyglykolether bzw. die Fettalkoholpolypropylenglykolether bzw. gemischte Fettalkoholpolyether,
- Anlagerungsprodukte von 2 bis 50 Mol Ethylenoxid und/oder 0 bis 5 Mol Propylenoxid an lineare und verzweigte Fettsäuren mit 6 bis 30 C-Atomen, die Fettsäurepolyglykolether bzw. die Fettsäurepolypropylenglykolether bzw. gemischte Fettsäurepolyether,
- Anlagerungsprodukte von 2 bis 50 Mol Ethylenoxid und/oder 0 bis 5 Mol Propylenoxid an lineare und verzweigte Alkylphenole mit 8 bis 15 C-Atomen in der Alkylgruppe, die Alkylphenolpolyglykolether bzw. die Alkylpolypropylenglykolether, bzw. gemischte Alyklphenolpolyether,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol^{®} - Typen (Cognis),
- alkoxilierte Triglyceride,
- alkoxilierte Fettsäurealkylester der Formel (Tnio-1)

   R¹CO-(OCH₂CHR²)_{w}OR³ (Tnio-1)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside,
- Zuckertenside vom Typ der Fettsäure-N-alkylpolyhydroxyalkylamide,

Die kationischen Tenside (Tkat) bilden die letzte Gruppe von Tensiden und wurden bereits zuvor ausführlich beschrieben.

Kationische, zwitterionische und/oder amphotere Tenside sowie deren Mischungen können erfindungsgemäß bevorzugt sein. Anionische Tenside werden insbesondere verwendet, wenn die erfindungsgemäßen Zusammensetzungen als Duschbäder verwendet werden sollen.

Die Tenside (T) werden in Mengen von 0,05 - 45 Gew.%, bevorzugt 0,1 - 30 Gew.% und ganz besonders bevorzugt von 0,5 - 25 Gew.%, bezogen auf das gesamte erfindungsgemäß verwendete Mittel, eingesetzt.

Erfindungsgemäß verwendbare Emulgatoren sind beispielsweise
- Ethylenoxid- und Polyglycerin-Anlagerungsprodukte an Methylglucosid-Fettsäureester, Fettsäurealkanolamide und Fettsäureglucamide,
- Partialester von Polyolen mit 3-6 Kohlenstoffatomen mit gesättigten Fettsäuren mit 8 bis 22 C-Atomen,
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- sowie der unter dem Stichwort "Tenside" beschriebenen Verbindungen.

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

Im folgenden werden einige Beispiele von besonders bevorzugten Polymeren beschrieben.

Bei den anionischen Polymeren handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder CoMonomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann. Besonders bevorzugt ist das Homopolymer der 2-Acrylamido-2-methylpropansulfon-säure, das beispielsweise unter der Bezeichnung Rheothik^{®}11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen. Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich. Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze^{®} QM im Handel erhältlich.

Die anionischen Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Geeignete nichtionogene Polymere sind beispielsweise:
- Vinylpyrrolidon/Vinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/VinylacetatCopolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) und Natrosol^{®}-Typen (Hercules) vertrieben werden.
- Stärke und deren Derivate, insbesondere Stärkeether, beispielsweise Structure^{®} XL (National Starch), eine multifunktionelle, salztolerante Stärke;
- Schellack
- Polyvinylpyrrolidone, wie sie beispielsweise unter der Bezeichnung Luviskol^{®} (BASF) vertrieben werden.

Die Polymere (P) sind in den erfindungsgemäß verwendeten Zusammensetzungen bevorzugt in Mengen von 0,01 bis 30 Gew.-%, bezogen auf die gesamte Zusammensetzung, enthalten. Mengen von 0,01 bis 25, insbesondere von 0,01 bis 15 Gew.-%, sind besonders bevorzugt.

Als Fettsäuren (Fatac) können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die lsostearinsäuren, wie die Handelsprodukte Emersol^{®} 871 und Emersol^{®} 875, und lsopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, lsotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge beträgt dabei 0,1 - 15 Gew.%, bezogen auf das gesamte Mittel. Bevorzugt beträgt die Menge 0,5 - 10 Gew.%, wobei ganz besonders vorteilhaft Mengen von 1 - 5 Gew.% sein können.

Als Fettalkohole (Fatal) können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, lsostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen von bevorzugt natürlichen Fettsäuren ab. Erfindungsgemäß einsetzbar sind ebenfalls solche Fettalkoholschnitte, die ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden. Die Fettalkohole werden in Mengen von 0,1 - 30 Gew.-%, bezogen auf die gesamte Zubereitung, bevorzugt in Mengen von 0,1 - 20 Gew.-% eingesetzt.

Als natürliche oder synthetische Wachse (Fatwax) können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Ein weiterer erfindungsgemäßer Wirkstoff in den erfindungsgemäßen Zusammensetzungen sind Proteinhydrolysate und/oder dessen Derivate (P).

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Pflanzenproteine und deren Hydrolysate sind beispielsweise Produkte auf der Basis von Weizen, Hafer, Reis, Mais, Kartoffeln, Moringa oder Soja. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda), Crotein^{®} (Croda) oder Puricare^{®} LS 9658 von der Fa. Laboratoires Sérobiologiques erhältlich.

Weitere erfindungsgemäß bevorzugte Proteinhydrolysate sind maritimen Ursprunges. Hierzu zählen beispielsweise Kollagenhydrolysate von Fischen oder Algen sowie Proteinhydrolysate von Muscheln bzw. Perlenhydrolysate. Beispiele für erfindungsgemäße Perlenextrakte sind die Handelsprodukte Pearl Protein Extract BG^{®} oder Crodarom^{®} Pearl.

Die Proteinhydrolysate (P) sind in den Zusammensetzungen in Konzentrationen von 0,001 Gew.% bis zu 20 Gew.%, vorzugsweise von 0,05 Gew.% bis zu 15 Gew.% und ganz besonders bevorzugt in Mengen von 0,05 Gew.% bis zu 5 Gew.% enthalten.

Die Wirkung der erfindungsgemäßen Zusammensetzungen kann weiterhin durch eine 2-Pyrrolidinon-5-carbonsäure und deren Derivate (J) gesteigert werden. Bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen 0,05 bis 10 Gew.%, bezogen auf das gesamte Mittel, besonders bevorzugt 0,1 bis 5, und insbesondere 0,1 bis 3 Gew.%.

Eine weitere bevorzugte Gruppe von Inhaltsstoffen der erfindungsgemäßen Zusammensetzungen mit dem erfindungsgemäßen Wirkstoffkomplex sind Vitamine, Provitamine oder Vitaminvorstufen.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.
- Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate. Die genannten Verbindungen des Vitamin B₅-Typs sind in den erfindungsgemäßen Mitteln bevorzugt in Mengen von 0,05 - 10 Gew.-%, bezogen auf das gesamte Mittel, enthalten. Mengen von 0,1 - 5 Gew.-% sind besonders bevorzugt.
- Vitamin B₆ (Pyridoxin sowie Pyridoxamin und Pyridoxal).

Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Schließlich ergeben sich durch die Verwendung von Pflanzenextrakten (L) in den erfindungsgemäßen Zusammensetzungen weitere Vorteile. Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Baldrian, Wiesenschaumkraut, Quendel, Schafgarbe, Thymian, Melisse, Hauhechel, Huflattich, Eibisch, Meristem, Ginseng, Kaffee, Kakao, Moringa, Ingwerwurzel, Wein, den Süßgräsern (Poaceaen), Litchi, insbesondere Litchi chinensis und ayurvedische Pflanzenextrakte wie Aegle Marmelos (Bilwa), Cyperus Rotundus (Nagar Motha), Emblica Officinalis (Amalki), Morida Citrifolia (Ashyuka), Tinospora Cordifolia (Guduchi), Santalum album, (Chandana), Crocus Sativus (Kumkuma), Cinnamonum Zeylanicum und Nelumbo Nucifera (Kamala) bevorzugt.

Als weiteren wesentlichen Inhaltsstoff können die erfindungsgemäßen Mittel Purin und/oder Derivat(e) des Purins enthalten. Unter Purin, den Purinen und den Purinderivaten sind erfindungsgemäß einige Vertreter besonders bevorzugt. Erfindungsgemäß bevorzugte kosmetische Mittel sind dadurch gekennzeichnet, daß sie Purin und/oder Purinderivat(e) der folgenden Verbindungen enthalten: Purin, Adenin, Guanin, Harnsäure, Hypoxanthin, 6-Purinthiol, 6-Thioguanin, Xanthin, Coffein, Theobromin, Theophyllin.

In haarkosmetischen Formulierungen hat sich insbesondere Coffein bewährt, das beispielsweise in Shampoos, Conditionern, Haarwässern und/oder Lotionen vorzugsweise in Mengen von 0,005 bis 0,25 Gew.-%, weiter bevorzugt von 0,01 bis 0,1 Gew.-% und insbesondere von 0,01 bis 0,05 Gew.-% (jeweils bezogen auf die Zusammensetzung) eingesetzt werden kann.

In den erfindungsgemäßen Mitteln sind unter geeigneten Biochinonen ein oder mehrere Ubichinon(e) und/oder Plastochinon(e) zu verstehen. Erfindungsgemäß besonders bevorzugt ist das Ubichinon der Formel mit n = 10, auch bekannt als Coenzym Q10.

Das oder die Biochinon(e) wird (werden) in den erfindungsgemäßen Mitteln - bezogen auf ihr Gewicht - in einer Menge von 0,0000005 bis 2%, bevorzugt in einer Menge von 0,000001 bis 1% und insbesondere in einer Menge von 0,00001 bis 0,5%.

Unter Polyhydroxyverbindungen sind organische Verbindungen mit mindestens zwei Hydroxygruppen zu verstehen. Insbesondere sind im Sinne der vorliegenden Erfindung hierunter zu verstehen:
- Polyole mit mindestens zwei Hydroxygruppen, wie beispielsweise Trimethylolpropan,
- Ethoxilate und/oder Propoxylate mit 1 bis 50 Mol Ethylenoxid und oder Propylenoxid der zuvor genannten Polyole,
- Kohlenhydrate, Zuckeralkohole und Zucker sowie deren Salze,
- insbesondere Monosaccharide, Disaccharide, Trisaccharide und Oligosaccharide, wobei diese auch in Form von Aldosen, Ketosen und/oder Lactosen,
- Aminodesoxyzucker, Desoxyzucker, Thiozucker, wobei diese auch in Form von Aldosen, Ketosen und/oder Lactosen.

Ganz besonders bevorzugt sind hierunter Monosaccharide mit 3 bis 8 C - Atomen, wie beispielsweise Triosen, Tetrosen, Pentosen, Hexosen, Heptosen und Octosen, wobei diese auch in Form von Aldosen, Ketosen und/oder Lactosen vorliegen können.

Weiterhin sind bevorzugt Oligosaccharide mit bis zu 50 Monomereinheiten, wobei diese auch in Form von Aldosen, Ketosen und/oder Lactosen vorliegen können.

Beispielhaft für die erfindungsgemäßen Polyole seien erwähnt Sorbit, Inosit, Mannit, Tetrite, Pentite, Hexite, Threit, Erythrit, Adonit, Arabit, Xylit, Dulcit, Erythrose, Threose, Arabinose, Ribose, Xylose, Lyxose, Glucose, Galactose, Mannose, Allose, Altrose, Gulose, Idose, Talose, Fructose, Sorbose, Psicose, Tegatose, Desoxyribose, Glucosamin, Galaktosamin, Rhamnose, Digitoxose, Thioglucose, Saccharose, Lactose, Trehalose, Maltose, Cellobiose, Melibiose, Gestiobiose, Rutinose, Raffinose sowie Cellotriose. Zusammenfassend sind erfindungsgemäße Zusammensetzungen bevorzugt, bei denen die Polyhydroxyverbindung ausgewählt ist aus Ethylenglycol, Propylenglycol, Polyethylenglycol, Polypropylenglycol, Glycerin, Glucose, Fructose, Pentaerythrit, Sorbit, Mannit, Xylit und ihren Mischungen.

Die erfindungsgemäßen Polyhydroxyverbindungen sind in den Zusammensetzungen in Konzentrationen von 0,01 Gew.% bis zu 20 Gew.%, vorzugsweise von 0,05 Gew.% bis zu 15 Gew.% und ganz besonders bevorzugt in Mengen von 0,1 Gew.% bis zu 10 Gew.% enthalten.

Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Zwei bevorzugte UV-Filter mit kationischen Gruppen sind die als Handelsprodukte erhältlichen Verbindungen Zimtsäureamidopropyl-trimethylammoniumchlorid (lncroquat^{®}UV-283) und Dodecyl-dimethylaminobenzamidopropyl-dimethylammoniumtosylat (Escalol^{®} HP 610).

Erfindungsgemäß besonders bevorzugt sind als UV - Filter diejenigen, welche zur Gruppe der substituierten Benzophenone, p-Aminobenzoesäureester, Diphenylacrylsäureester, Zimtsäureester, Zimtsäureamidopropyl-trialkylammoniumchloride, Salicylsäureester, Octocrylene, Benzimidazole, Dodecyl-dimethylaminobenzamidopropyl-dimethylammoniumtosylate und o-Aminobenzoesäureester zugehörig sind. Als höchst bevorzugte UV - Filter sind zu nennen: Benzophenone-4, Octocrylen, Zimtsäureamidopropyl-trimethylammoniumchlorid und Dodecyl-dimethylaminobenzamidopropyldimethylammoniumtosylat.

Die UV-Filter (I) sind in den erfindungsgemäß verwendeten Mitteln üblicherweise in Mengen 0,1-5 Gew.-%, bezogen auf das gesamte Mittel, enthalten. Mengen von 0,4-2,5 Gew.-% sind bevorzugt.

Es kann erfindungsgemäß bevorzugt sein, das erfindungsgemäße Mittel im Rahmen einer Farbveränderung der Haare, zu verwenden. Insbesondere die oxidative Farbveränderung ist dabei bevorzugt, da die Pflegewirkung der erfindungsgemäßen Mittel auch bei Gegenwart eines Oxidationsmittels hervorragend ist.

Ferner können die kosmetischen Mittel weitere Wirk-, Hilfs- und Zusatzstoffe enthalten, wie beispielsweise
- Farbstoffe zum Anfärben des Mittels,
- Antischuppenwirkstoffe wie Piroctone Olamine, Zink Omadine und Climbazol,
- Komplexbildner wie EDTA, NTA, β-Alanindiessigsäure und Phosphonsäuren,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher verwiesen.

Ein weiterer Gegenstand der vorliegenden Erfindung ist ein Verfahren, insbesondere ein kosmetisches Verfahren, zur Vitalisierung von keratinischen Fasern, Haarverdickung, Anregung der Keratinsynthese, Anregung des Energiestoffwechsel in Haarfollikeln, Aktivierung von Haarfollikeln, Förderung oder Verstärkung des Wuchses von keratinischen Fasern, zur Restrukturierung keratinischer Fasern bzw. zur Haarkonditionierung, **dadurch gekennzeichnet, dass** man eine erfindungsgemäße Zusammensetzung, auf die Haare bzw. die behaarte Haut aufbringt.

Die folgenden Beispiele sollen den Gegenstand der vorliegenden Erfindung erläutern ohne ihn jedoch zu beschränken.

### Beispiele und Wirkungsnachweise

### Alle Mengenangaben sind, soweit nicht anders vermerkt, Gewichtsteile.

### 1. Wirkungsnachweis der Restrukturierung

### Vorbehandlung

Strähnen der Fa. Alkinco (1,0 g, Code 6634) wurden mit 2,0 g einer Lösung aus 10,0 Gew. % Natriumlaurylethersulfat (2EO) für 2 Minuten shampooniert. Anschließend wurde 1 Minute unter fließendem Wasser von 23 °C ausgespült und die Haarsträhne mit einem Haarföhn getrocknet. Es folgte eine herkömmliche Dauerwellbehandlungen mit 2,0 g des Handelsproduktes Poly Lock-Normale Dauerwelle. Im Rahmen dieser Dauerwellbehandlung wurden die Fasern in einem ersten Schritt für 40 Minuten bei Raumtemperatur der Reduktionslösung (enthaltend 7,9 Gew.-% Thioglykolsäure) ausgesetzt, mit reinem Wasser mit einer Temperatur von 23 °C für 1 Minute unter fließendem Wasser gespült, mit einem Handtuch getrocknet und anschließend bei Raumtemperatur für 10 Minuten mit 2,0 g einer Oxidationslösung fixiert. Die Oxidationslösung enthielt 2,6 Gew.-% Wasserstoffperoxid. Nach der oxidativen Behandlung wurden die Fasern wiederum für 1 Minute unter fließendem Wasser bei 23 °C gespült und anschließend mit einem Haarföhn getrocknet. Die Dauerwellbehandlung wurde insgesamt unter identischen Bedingungen zweimal durchgeführt.

### Nachbehandlung

Die Strähnen wurden jeweils bei einer Temperatur von 32°C 30 Minuten in eine wäßrige Lösung der jeweiligen Wirkstoffe bei einem pH - Wert von 7, welcher mit Natronlauge oder Salzsäure eingestellt wurde, getaucht. Nach der Behandlung mit der jeweiligen Wirkstoffzusammensetzung wurde jede Haarsträhne 1 Minute mit klarem Wasser gespült, an der Luft getrocknet und 16 h ruhen gelassen.

### Nachweis der haarstrukturierenden Wirkung mittels HP-DSC

Mittels einer DSC-Analyse (Perkin Elmer DSC-7) wurden die folgenden in Tabelle 1 dargestellten Schmelzpunkte ermittelt. Eine genaue Beschreibung der Methode findet sich beispielsweise in der DE 196 173 95 A1.

| Wirkstoff bzw. Wirkstoffkomplex | Schmelzpunkt in °C |
|---|---|
| 1. unbehandeltes Haar (Kontrolle) | 151,9 |
| 2. kaltgewelltes Haar ohne Wirkstoff | 147,3 |
| 3. 0,2 % Carnitintartrat, 0,2% Taurin und 0,05% Echinaceae purp | 154,2 |
| 4. 0,1% Taurin und 0,01% Echinaceae purp. | 152,6 |
| 5. 0,2% Carnitintartrat, 0,1% Taurin und 0,01% Echinaceae purp. | 153,4 |

Das Signifikanzniveau des erfindungsgemäßen Wirkstoffkomplexes unter 3. gegenüber dem Wert für unbehandeltes Haar beträgt >99%.

### 2. Wirkungsnachweis der Reduktion des Haarbruches

### Vorbehandlung

Strähnen der Fa. Kerling (1,0 g, European natural hair 6/0)mit einer Länge von 12 cm wurden mit 2,0 g einer Lösung aus 10,0 Gew. % Natriumlaurylethersulfat (2EO) für 5 Minuten shampooniert. Anschließend wurde 3 Minute unter fließendem Wasser von 23 °C ausgespült und gleichzeitig gekämmt.

### Nachbehandlung / Anwendung der Wirkstoffe:

Anschließend wurden die Haarsträhnen 15 Minuten in die wässrige Lösung der Wirkstoffe bei ca. 23°C getaucht. Anschließend wurden die Haarsträhnen 5 Minuten unter fließendem Wasser mit einer Temperatur von 32°C und einem Durchfluß von 0,5 I Wasser pro Minute gespült und wiederum gleichzeitig gekämmt. Daran anschließend wurden die Strähnen bei 25°C und 25% rel. Feuchte für mindestens 24 h konditioniert.

### Nachweis der Reduktion des Haarbruches:

Jede Haarsträhne, die wie zuvor behandelt wurde, wurde bei 25°C und 25% rel. Feuchte mit einem feinzähnigem, Hartgummikamm der Bezeichnung HERCULES Sägemann 20.000 mal ausgekämmt. Der hierbei aufgetretene Haarbruch wurde unterhalb des Kammes in einem Behälter aufgefangen. Im Anschluß daran wurde von den Haarsträhnen 1,5 cm gemessen von der untersten Spitze der Strähne abgeschnitten und je Strähne gesammelt. Diese Mengen an Haar wurden genau ausgewogen und zueinander in Beziehung gesetzt.

| Wirkstoff bzw. Wirkstoffkomplex | Haarbruch nach 20.000 Kämmcyclen in % |
|---|---|
| 1. unbehandeltes Haar (Kontrolle) | 11 |
| 2. 0,2 % Carnitintartrat, 0,2% Taurin und 0,05% Echinaceae purp | 0,3 |

Das Signifikanzniveau des erfindungsgemäßen Wirkstoffkomplexes gegenüber dem Wert für unbehandeltes Haar beträgt >99%. Die Reduktion des Haarbruches gegenüber der unbehandelten Haarprobe beträgt 97%. Somit ist das Haar, welches mit der erfindungsgemäßen Zusammensetzung behandelt wurde, statistisch signifikant deutlich vor Haarbruch geschützt.

### 3. Weitere Formulierungsbeispiele:

Die folgenden Rezepturen wurden unter Anwendung bekannter Herstellungsverfahren bereitgestellt. Dabei wurden unter anderem nachstehende Handelsprodukte als Rohstoffe verwendet:

| Shampoo | | | | |
|---|---|---|---|---|
| | S-1 | S-2 | S-3 | S-4 |
| Texapon N70 | 15,0 | 15,0 | 15,0 | 15,00 |
| Incromine BB | 0,3 | 0,5 | 0,8 | 1,0 |
| Antil 141 L | | | 1,0 | 1,0 |
| Gluadin WQ | | | 0,3 | 0,3 |
| DC 193 Fluid | | | 0,5 | 0,5 |
| Quaternium-91 | 0,1 | 0,1 | 0,1 | 0,1 |
| PPG-3 Benzyl Ether Myristate | 0,1 | 0,1 | 0,1 | 0,1 |
| Extrakt aus Echinaceae purp. | 0,1 | 0,1 | 0,1 | 0,1 |
| Carnitintartrat | 0,1 | 0,1 | 0,1 | 0,1 |
| Dehyquart L 80 | | 1,0 | | 1,0 |
| Rewoteric RTM 50 | 1,0 | | 1,0 | |
| Abilquat 3272 | 0,8 | 1,5 | 0,5 | 1,0 |
| Lamesoft PO 65 | 0,3 | 0,3 | - | - |
| Dow Corning 200, 60000 cSt | - | 0,1 | 0,1 | - |
| Dow Corning 200 Fluid, 0,65 cSt | 0,1 | 0,1 | 0,1 | 0,1 |
| Natriumbenzoat | 0,5 | 0,5 | 0,5 | 0,5 |
| Dehyton PS | 6,0 | 6,0 | 6,0 | 6,0 |
| Salicylsäure | 0,2 | 0,2 | 0,2 | 0,2 |
| Euperlan PK 3000 AM | 2,0 | 2,0 | | |
| D-Panthenol | 0,1 | 0,1 | | |
| Nikotinsäureamid | 0,1 | 0,1 | | |
| Cetiol HE | 0,3 | 0,3 | 1,5 | 1,5 |
| Cremophor CO 40 | | | 2,0 | 2,0 |
| Polyquaternium 10 | 0,2 | 0,2 | 0,5 | 0,5 |
| Natriumchlorid | 1,5 | 1,5 | 0,3 | 0,3 |
| Litchiderm LS 9704 | 0,1 | 0,5 | 0,05 | 0,2 |
| Ectoin | 0,1 | 0,5 | 0,3 | 0,3 |
| Taurin | 0,5 | 1,0 | 0,25 | 0,75 |
| Extrapone White Tea GW | 0,1 | 0,5 | 0,05 | 0,2 |
| Wasser | Ad 100 | Ad 100 | Ad 100 | Ad 100 |

| Spülung | | | | |
|---|---|---|---|---|
| | Sp-1 | Sp-2 | Sp-3 | Sp-4 |
| Cutina GMS-V | 0,3 | 0,3 | 0,2 | 0,2 |
| Dehyquart F 75 | 3,8 | 0,8 | 1,0 | 1,0 |
| Lanette O | 4,0 | 4,0 | 3,0 | 3,0 |
| Eumulgin B2 | 0,2 | 0,2 | 0,3 | 0,3 |
| Varisoft W 575 PG | | | 4,0 | 4,0 |
| Quaternium-91 | 3,5 | 4,0 | 2,5 | 3,0 |
| PPG-3 Benzyl Ether Myristate | 0,5 | 0,5 | 0,8 | 0,8 |
| Incroquat Behenyl HE | 2,0 | 2,0 | 2,0 | 2,0 |
| Dow Corning 200, 60000 cSt | 1,0 | 0,5 | 1,5 | 0,3 |
| Dow Corning 200 Fluid, 0,65 cSt | 0,5 | 0,5 | 0,3 | 0,1 |
| Extrakt aus Echinacea purp. | 0,1 | 0,1 | 0,1 | 0,1 |
| Tego Amid S 18 | 1,5 | 2,0 | 0,3 | 0,3 |
| Empigen CSC | 0,5 | 0,75 | 1,5 | 2,0 |
| Salcare SC 96 | 0,4 | 0,3 | 0,6 | 0,6 |
| Zitronensäure | | | 0,4 | 0,4 |
| D-Panthenol | | | 0,2 | 0,2 |
| Ajidew NL 50 | | | 1,0 | 1,0 |
| Dehyquart A-CA | 3,0 | 3,0 | | |
| Milchsäure | 0,5 | 0,5 | | |
| Phenoxyethanol | 0,4 | 0,4 | 0,3 | 0,3 |
| Wacker Belsil ADM 8020 VP | 0,9 | 0,9 | | |
| Carnitintartrat | 0,1 | 0,3 | 0,3 | 0,3 |
| Taurin | 0,1 | 0,3 | 0,5 | 0,3 |
| Wasser | Ad 100 | Ad 100 | Ad 100 | Ad 100 |

## Patentansprüche

1. Kosmetische Zusammensetzung zur Reduktion des Bruches keratinischer Fasern, enthaltend in einem kosmetischen Träger
a) mindestens ein Betain der Formel (Bet-I),
b) mindestens Taurin oder ein Taurinderivat und
c) mindestens einen Wirkstoff erhältlich aus Pflanzen der Gattung Echinacea.

2. Kosmetische Zusammensetzung zur Reduktion des Bruches keratinischer Fasern nach Anspruch 1, **dadurch gekennzeichnet, dass** das Betain der Formel (Bet-I) in Form eines Mischsalzes verwendet wird.

3. Kosmetische Zusammensetzung zur Reduktion des Bruches keratinischer Fasern nach einem der Ansprüche 1 bis 2, **dadurch gekennzeichnet, dass** das Betain der Formel (Bet-I) ausgewählt ist aus Carnitin, Histidin, N,N,N-trimethylglycin (Betain), Cholin und Dimethylprolin.

4. Kosmetische Zusammensetzung zur Reduktion des Bruches keratinischer Fasern nach einem der Ansprüche 1 bis 3, **dadurch gekennzeichnet, dass** das Betain der Formel (Bet-I) in Form eines Mischsalzes verwendet wird und die Säure ausgewählt ist aus Fumarsäure, Zitronensäure, Milchsäure, Weinsäure, Histidin, Arginin, Lysin, Ornithin und Citrullin.

5. Kosmetische Zusammensetzung zur Reduktion des Bruches keratinischer Fasern nach einem der Ansprüche 1 bis 4, **dadurch gekennzeichnet, dass** der Inhaltsstoff a) Carnitin - Tartrat ist.

6. Kosmetische Zusammensetzung zur Reduktion des Bruches keratinischer Fasern nach einem der Ansprüche 1 bis 5, **dadurch gekennzeichnet, dass** der Inhaltsstoff b) Taurin ist.

7. Kosmetische Zusammensetzung zur Reduktion des Bruches keratinischer Fasern nach einem der Ansprüche 1 bis 6, **dadurch gekennzeichnet, dass** der Wirkstoff erhältlich aus Pflanzen der Gattung Echinacea ein Presssaft aus Echinacea purpurea ist.

8. Kosmetische Zusammensetzung zur Reduktion des Bruches keratinischer Fasern nach einem der Ansprüche 1 bis 7, **dadurch gekennzeichnet, dass** weiterhin mindestens ein Esteröl enthalten ist.

9. Kosmetisches Verfahren zur Reduktion des Bruches keratinischer Fasern, **dadurch gekennzeichnet, dass** eine Zusammensetzung gemäß einem der Ansprüche 1 bis 8 auf die keratinischen Fasern aufgebracht wird und nach einer Einwirkzeit von 10 Sekunden bis zu 60 Minuten wieder ausgespült wird.

10. Kosmetisches Verfahren zur Reduktion des Bruches keratinischer Fasern, **dadurch gekennzeichnet, dass** eine Zusammensetzung gemäß einem der Ansprüche 1 bis 8 auf die keratinischen Fasern aufgebracht wird und auf den keratinischen Fasern bis zur nächsten Wäsche der keratinischen Fasern verbleibt.
